(19) Europäisches Patentamt / European Patent Office / Office européen des brevets

(11) **EP 1 771 549 B1**

(12) **EUROPÄISCHE PATENTSCHRIFT**

(45) Veröffentlichungstag und Bekanntmachung des Hinweises auf die Patenterteilung:
**13.05.2009 Patentblatt 2009/20**

(21) Anmeldenummer: **05763068.3**

(22) Anmeldetag: **20.07.2005**

(51) Int Cl.:
*C12N 5/06* (2006.01)       *C12N 5/08* (2006.01)
*C07K 16/28* (2006.01)       *C12N 5/20* (2006.01)

(86) Internationale Anmeldenummer:
**PCT/EP2005/053520**

(87) Internationale Veröffentlichungsnummer:
**WO 2006/013159 (09.02.2006 Gazette 2006/06)**

(54) **VERFAHREN ZUR ISOLIERUNG UND KULTIVIERUNG VON NEURALEN STAMMZELLEN**

METHOD FOR ISOLATING AND CULTURING NEURAL STEM CELLS

PROCÉDÉ POUR ISOLER ET METTRE DES CELLULES SOUCHES NEURALES EN CULTURE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **31.07.2004 DE 102004037234**

(43) Veröffentlichungstag der Anmeldung:
**11.04.2007 Patentblatt 2007/15**

(73) Patentinhaber: **Ruhr-Universität Bochum
44801 Bochum (DE)**

(72) Erfinder:
• **FAISSNER, Andreas
44801 Bochum (DE)**
• **SIRKO, Svetlana
44359 Dortmund (DE)**
• **HOLST, Alexander von
44225 Dortmund (DE)**

(74) Vertreter: **Helbing, Jörg
Patentanwälte
Von Kreisler-Selting-Werner
Postfach 10 22 41
50462 Köln (DE)**

(56) Entgegenhaltungen:
**WO-A-20/04007697       WO-A2-00/52143**

• **FAISSNER ANDREAS ET AL: "Isolation of a neural chondroitin sulfate proteoglycan with neurite outgrowth promoting properties" JOURNAL OF CELL BIOLOGY, Bd. 126, Nr. 3, 1994, Seiten 783-799, XP002372969 ISSN: 0021-9525 in der Anmeldung erwähnt**
• **"Products for Life Science - Research [CATALOGUE] - Reagents and Kits for Antibody Detection - Peroxidase Conjugates (pages 291-293)" 2000, SIGMA ALDRICH CHEMIE BV , ZWIJNDRECHT, NEDERLAND , XP002373337 Seite 293**
• **CLEMENT ALBRECHT M ET AL: "The DSD-1 carbohydrate epitope depends on sulfation, correlates with chondroitin sulfate D motifs, and is sufficient to promote neurite outgrowth" JOURNAL OF BIOLOGICAL CHEMISTRY, Bd. 273, Nr. 43, 23. Oktober 1998 (1998-10-23), Seiten 28444-28453, XP002372970 ISSN: 0021-9258 in der Anmeldung erwähnt**
• **WU ET AL: 'The tissue plasminogen activator (tPA)/plasmin extracellular proteolytic system regulates seizure-induced hippocampal mossy fiber outgrowth through a proteoglycan substrate' JOURNAL OF CELL BIOLOGY Bd. 148, Nr. 6, 20 März 2000, Seiten 1295 - 1304**

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft ein Verfahren zur Isolierung und Kultivierung von neuralen Stammzellen.

**Hintergrund der Erfindung**

Seit den ersten Studien, die über erfolgreiche Isolierung neuraler Stammzellen aus verschiedenen Regionen des embryonalen ZNS berichteten (Temple, S., Nature 340:471-473 (1989); Cattaneo, E. et al., Nature 347:762-765 (1990); Kilpatrick, T. J. et al., Neuron. 10:255-265 (1993)) beschäftigt die Neurowissenschaft die fundamentale Frage, ob die neuralen Stammzellen eine kleine Population des Neuroectoderms darstellen, oder ob alle Zellen des Neuralrohres Stammzellen sind.

Mit der Isolierung adulter neuraler Stammzellen aus zwei neurogenen Regionen des adulten ZNS, dem Hippokampus und der subventrikulärer Zone, begann ein neues Kapitel der Neurobiologie (Reynolds, B. A. et al., J. Neurosci. 12:4565-4574 (1992); Lois, C. et al., Science 255:1707-1710 (1993)). Dabei stand nicht nur die Charakterisierung der neuralen Stammzellen im Vordergrund, sondern auch das Verständnis der zeitlichen und positionellen Signalübertragungssysteme bei diesen Zellen.

Die Beobachtungen und Ergebnisse dieser Studien bildeten die Grundlage für die initialen Identifikationscharakteristika der neuralen Stammzellen nach der Isolierung. Man stellte fest, dass *in vitro* aus einem großen Anteil dieser Stammzellen in serumfreiem Medium multizelluläre Neurosphären kultiviert werden können und dass aus isolierten neuralen Stammzellen Neurone und Gliazellen differenzieren (McKay, R. D., Science 276:66-71 (1997); Rao, M. S., Anat. Rec. 257:137-148 (1999); Gage, F. H., Science 287:1433-1438 (2000)). Die Bildung primärer Neurosphären in serumfreiem Medium zeigte, dass die neuralen Stammzellen auch *in vitro* unter Gabe von Wachstumsfaktoren ihr Proliferationspotential beibehalten. Auch nach Rekultivierung sind diese Zellen des weiteren in der Lage, sekundäre Neurosphären zu bilden. Außerdem sind neurale Stammzellen durch ihren pluripotenten Charakter, der zur Differenzierung unterschiedlicher neuraler Zelltypen führt, gekennzeichnet.

Die Spezifität der neuralen Stammzellen wird von räumlichen und zeitlichen Signalinformationen beeinflusst. Aus verschiedenen Regionen des ZNS isolierte Stammzellen bilden multizelluläre Neurosphären, wobei jede dieser Zellen regionspezifische Marker exprimiert. Die regulatorischen Sequenzen kontrollieren die regionspezifische Expression von Transkriptionsfaktoren wie z.B. Sox2. Dieser Faktor wird z. B. von Stammzellen aus dem Telencephalon, nicht aber aus dem Rückenmark, exprimiert (Zappone, M. et al., Development 127:2367-2382 (2000)).

Die Differenzierung neuraler Stammzellen ist zwar durch ihr Potential und ihren Phänotyp festgelegt, wird aber entscheidend von Signalmolekülen, wie z. B. Fibroblast Growth Factor (FGF), Bone Morphogenetic Protein (BMP) und Noggin, schon während der neuralen Induktion reguliert (Wolpert, L. et al., Dev. Genet. 15:485-490 (1994); Altman & Brivanlou, Int. Rev. Cytol. 203:447-482 (2001)).

Die Präzision der Zelldifferenzierung in Neurone und Gliazellen während der embryonalen Entwicklung ist eine entscheidende Voraussetzung für die Organisation einer intakten Zellarchitektur des neuralen Gewebes. Die zeitlich streng determinierten zellulären Entwicklungsprozesse, wie Stammzellproliferation und Differenzierung der Vorläuferzellen mit folgender Migration der ausdifferenzierten Zellen zu ihrer endgültigen Lokalisation, werden durch Kombination von intrinsischen und extrazellulären Signalen reguliert.

Das Studium neuraler Stammzellen ist nicht nur für das Verständnis der Entwicklung des Nervensystems von hoher Bedeutung, sondern auch wegen ihres therapeutischen Potentials in der Behandlung neurodegenerativer Krankheiten.

Bedingt durch die vielseitige Anwendbarkeit der neuralen Stammzellen wurden Wege gesucht, um diese in zureichender Qualität und Reinheit herzustellen.

WO 00/52143 beschreibt die Isolation von Vorläuferzellen des peripheren Nervensystems aus der vorübergehenden embryonalen Struktur der Neuralleiste. Es wird eine immunologische Sortierungsmethode benutzt, die auf einem monoklonalen Antikörper gegen den niederaffinen NGF-Rezeptor p75 beruht.

US 5,928,947, US 5,654,183, US 5,672,499 und US 5,693,482 beschreiben Isolierung, Klonierung und einen Bioassay zur Charakterisierung von Vorläuferzellen des peripheren Nervensystems aus der Neuralleiste.

DE-A-102 42 337 betrifft die Sortierung neuronaler Vorläuferzellen und ist damit auf Vorläufer von Nervenzellen (Neuronen) beschränkt. Die Sortierung erfolgt über das CD133-Antigen.

US-A-2002/0168767 beschreibt eine Methode zur Reinigung von menschlichen neuroepithelialen Vorläuferzellen aus Kulturen von Fötalzellen durch eine aufwändige 3-stufige Immunodepletion der nicht-neuroepithelialen Vorläuferzellen.

Die in diesen Schriftstücken beschriebenen Verfahren sind jedoch nicht zufriedenstellend hinsichtlich ihrer Anwendbarkeit auf Vorläuferzellen des Zentralnervensystems und ermöglichen keine Anreicherung neuraler Stammzellen im präparativen Maßstab mit ausreichender Reproduzierbarkeit.

Obwohl die neuralen Stammzellen schon in frühen Entwicklungsstadien ein regionalspezifisches Genexpressionsmuster (Tropepe, V. et al., Dev. Biol. 2-08:166-188 (2000)) und Zellmigrationsverhalten aufweisen, sind die entscheidenden lokalen regulatorischen Prozesse über die Zellinteraktion beeinflusst. Die Zell-ZellInteraktion kann direkt über die kontaktierenden Zelloberflächen zustande kommen oder wird über molekulare Bestandteile der gewebespezifischen extrazellulären Matrix (ECM) vermittelt. Die Moleküle der ECM regulieren die entscheidenden Schritte der Entwicklung des ZNS und beeinflussen die Kontrolle der zellulären Differenzierung und Migration (Brown, M. et al., The Developing Brain, Oxford Univ. Press I nc., London, New York (2001)).

Die an Proteinen und Kohlenhydraten reichhaltige extrazelluläre Matrix (ECM) des neuralen Gewebes kann generell in drei Komponenten, fibrilläre Elemente (z.B. Elastin), modulare Proteine (z.B. Fibronektin und Laminin), und polymere Makromoleküle (z.B. Glykosaminoglykane) unterteilt werden.

Die größte Fraktion der aus Heparansulfaten oder Chondroitinsulfaten bestehenden Glykosaminoglykane (GAG) bilden die Chondroitinsulfat proteoglykane (CSPG). Diese biochemisch heterogene Gruppe ist nicht nur während der embryonalen Entwicklung funktionell signifikant, sondern auch entscheidend an neuralen Regenerationsprozessen beteiligt (Lander, A. D., Curr. Opin. Neurobiology 3:716-723 (1993); Bandtlow & Zimmermann, Phys. Rev. 80:1267-1290 (2000)). Ein wichtiges CSPG des Nervengewebes ist Phosphacan.

WO 95/20397 beschreibt Phosphacan, dessen Herstellung und dessen Verwendung zur Gewinnung von Rezeptoren und Erkennungsmolekülen sowie zur Isolierung von gereinigter oder gentechnisch produzierter Phosphatase, zur Förderung der Regeneration des Nervensystems, zur Herstellung von monoklonalen oder polyklonalen Antikörpern gegen Phosphacan und zur Diagnose und Beurteilung von Hirntumoren.

Phosphacan, ein sekretiertes Ratten-CSPG, wurde in Studien an Hirngewebe von Ratten hinsichtlich seiner molekularen, biochemischen und immunochemischen Eigenschaften charakterisiert und beschrieben. DSD-1-PG, eines der CSPG im neuralen Gewebe bei Mäusen, ist dem Phosphacan aus neuralen Gewebe der Ratten hinsichtlich der Peptidsequenzen und deren Anordnung, der Domänenkomposition und der Anzahl von proteolytischen Fragmenten homolog (Faissner, A. et al., J. Cell. Biol. 126:783-799 (1994); Garwood, J. et al., J. Neurosci 19:51 - 64 (1999)). Phosphacan spielt eine Rolle bei Neuron-Glia-Interaktionen, die an neuraler Differenzierung, Myelinisierung und Regeneration beteiligt sind (Garwood, J. et al., J. Biol. Chem. 278:24164-14173 (2003)).

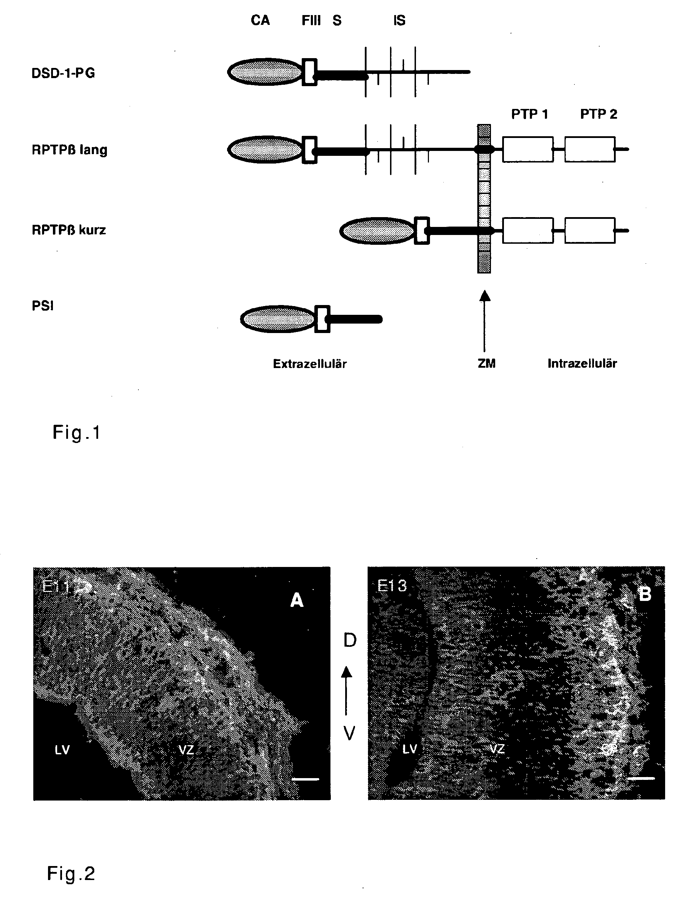

DSD-1-PG/Phosphacan hat eine molekulare Masse zwischen 800 bis 1000 kD und zeigt einen für Proteoglykane typischen Aufbau aus einem Polypeptidgerüst mit mehreren Polysaccharidketten (Fig. 1).

Das Aminoende des Proteingerüstes dieses Proteoglykans bilden eine Carbo-anhydrasedomäne (CA) und eine Fibronectin Typ III Domäne (FIII). Darauf folgt die Spacer (S) Region und die intervenierende Peptidregion (IS). IS ist stark mit Chondroitinsulfat-Glycosaminoglykanketten (CS GAG) glykosyliert, die kovalent an das Peptidgerüst gebunden sind. Die CS GAG sind Polymere aus 50 bis 100 sich wiederholenden Disacchariden, D-Glukuronsäure und N-Acetyl-Galactosamin. Der heterogene Charakter der GAG-Strukturen ist durch die variierte spezifische Komposition dieser Dimere sowie deren Sulfatierung hervorgerufen. Die CS GAG von DSD-1-PG/Phosphacan bestehen aus CS-A, CS-D und überwiegend CS-C Kettenmotiven. Biochemische Studien zeigten, dass die CS-D-Ketten reich an 14 linear angeordneten Disacchariden sind und in Abhängigkeit von der Sulfatierung diese Oligosaccharide mit Hilfe des monoklonalen 473HD Antikörpers identifiziert werden können (Clement, A. M. et al., J. Biol. Chem. 273:28444-28453 (1998)).

Das DSD-1-Epitop wurde mit Hilfe des monoklonalen Antikörpers (Mab) 473HD entdeckt und zunächst in Mauszellen beschrieben. Die Sensitivität des Epitops gegen das Enzym Chondroitinase ABC verwies darauf, dass die Struktur in die Klasse der Chondroitinsulfate einzuordnen ist (Faissner, A., Soc. Neurosci. Abstr. 14:920 (1988); Faissner, A. et al., J. Cell. Biol. 126:783-799 (1994)). Eine weitergehende biochemische Analyse hat in der Folgezeit herausgestellt, dass das Epitop des Mab 473HD das Chondroitinsulfat-Motiv D enthält, das in Sequenz mit dem Chondroitinsulfat A, z.B. in der Sequenz -A-D- auftritt. Ferner schließt das Epitop voraussichtlich auch ein Iduronsulfat-Motiv ein und ist von der Sulfatierung der Glykosaminoglykan-Kohlehydratketten abhängig (Clement, A.M. et al., J. Biol. Chem. 273: 28444-28453 (1998); Hikino, M. et al., Biol. Chem. 278:43744-43754 (2003); Bao, X. et al., J. Biol. Chem. 280:9180-9191 (2005)). Die vorliegenden Daten untermauern, dass das Mab 473HD-Epitop sich strukturell von den Epitopen anderer, gegen Chondroitinsulfate gerichteter monoklonaler Antikörper, z.B. des CS-56 oder des Mo-225 unterscheidet. Damit stellt es nach heutiger Kenntnis eine einzigartige Struktur dar, die eigene, unverwechselbare Expressionsmuster und funktionelle Eigenschaften aufweist (Ito, Y. et al., Glycobiology 15:593-603 (2005)). Histologische Untersuchungen zeigten das Epitop in diskreten Regionen während der Entwicklung des Hühnerembryos (Heyman et al., Dev. Dynam. 204:301 -315, (1995)) und in sog. perineuronalen Netzen des ZNS der Ratte (Wintergerst, E.S. et al., Int. J. Dev. Neurosci. 14:249-256 (1996)). Im Rahmen der biochemischen Charakterisierung stellte sich heraus, dass DSD-1 auch in Präparationen von Hai-Knorpel nachweisbar ist (Clement, A.M. et al., J. Biol. Chem. 273:28444-28453 (1998)). Die Expression in Amphibien wird durch aktuellen Untersuchungen untermauert, die DSD-1 in Danio rero (Zebrafisch) identifizierten (M. Bastmeyer, A. Faissner, unveröffentlichte Beobachtungen).

Zusätzlich zu den Chondroitinsulfat-GAG-Ketten kann das DSD-1-PG/Phosphacan auch Keratansulfat-GAG-Ketten und andere, N-verknüpfte Zuckerreste, wie das HNK-1-Epitop und Lewis-X, aufweisen (Meyer-Puttliz, B. et al., J. Neurochem. 65:2327-2337 (1995); Allendoerfer, K. L. et al., Dev. Biology 211:208-219 (1999)).

Dieser molekulare Aufbau des DSD-1-PG/Phosphacans ist strukturell äquivalent zum extrazellulären Anteil der langen Variante der Rezeptorproteins Tyrosinphosphatase, RPTPβ (Krueger, N. X., Saito, H., Proc. Natl. Acad. Sci. USA 89:7417-7421 (1992); Canoll, P. D. et al., Brain Res. 75:293-298 (1993); Maurel, P. et al., Proc. Natl. Acad. Sci. USA 91:2512-2516 (1994)). Die extrazellulären Domänen von RPTPβ sind durch eine Transmembrandomäne mit zwei intrazellulären Tyrosinphosphatasedomänen verbunden. Man unterscheidet die lange Isoform (RPTPβ-long), die eine dem DSD-1-PG/Phosphacan identische extrazelluläre Region aufweist, von der kurzen Isoform des RPTPβ (RPTPβ-short), die, bis auf 850 fehlende Aminosäuren der IS-Region zwischen S und Transmembrandomäne, den gleichen Aufbau wie die RPTPβ-long Isoform hat. RPTP-β ist auch unter der Bezeichnung RPTP-ζ bekannt und wird daher auch RPTP-beta/zeta genannt.

In mehreren Studien an neuralem Gewebe wurde ein Expressionsprofil von DSD-1-PG/Phosphacan/RPTPβ erstellt. Die ziemlich eindeutige Lokalisation des DSD-1-PG/Phosphacan auf glialen Vorläufern und ausdifferenzierten Gliazellen wurde nach Beginn der Gliogenese, d. h. ab ca. E15-16 bei Mäusen, festgestellt. Gleichzeitig wurde die Expression des DSD-1-PG/Phosphacan in Regionen aktiver Zellproliferation auch in frühen Entwicklungsstadien wie E13 beobachtet (Engel, M. et al., J. Comp. Neurobiol. 366:34-43 (1996); Meyer-Puttlitz, B. et al., J. Comp. Neurobiol. 366:44-54 (1996)). In diesen embryonalen Stadien weist das Neuroepithelium neben neuralen Stammzellen auch radiale Gliazellen und erste Neuroblasten auf, aber keine glialen Vorläufer.

So wird das DSD-1-PG/Phosphacan/RPTPβ in den Regionen aktiver Zellproliferation, wie dem Neuroepithelium der ventrikulären Zone und der ependymalen Schicht entlang des Zentralkanals des Rückenmarkes, exprimiert (Ivanova, A. et al., Gene Expression Patterns 4:161-166 (2004); Kabos, P. et al., Biochem. Biophys. Res. Comm. 318:955-963 (2004)). Dabei zeigten die immunhistologischen Analysen und *in situ* Hybridisierungen ein Expressionsmuster entlang radial angeordneter Zellfortsätze in der ventrikulären Zone, wo sich während der embryonalen Entwicklung die neuralen Stammzellen und radiale Gliazellen befinden. Dadurch ließ sich zwar vermuten, dass diese Expression auf den radialen Gliazellen lokalisiert ist (Canoll, P. D. et al., Brain Res. 75:293-298 (1993), Canoll, P. D. et al., J. Neurosci. Res. 44: 199-219 (1996)) und eventuell Konsequenzen für die Zellmigration hat (Engel, M. et al., J. Comp. Neurol. 366:34-43 (1996); Meyer-Puttlitz, B. et al., J. Comp. Neurol. 366:44-54 (1996)), aber direkte Beweise für die Expression von DSD-1-PG/Phosphacan/RPTPβ auf den radialen Gliazellen sind zur Zeit nicht bekannt.

Bei späteren Entwicklungsstadien primärer cerebellarer Kulturen und in sekundär angereicherten glialen Zellkulturen wurde die Lokalisation des DSD-1-PG/Phosphacans an den glialen Vorläuferzellen, den O4-positiven und O1-negativen Oligodendrozyten festgestellt (Faissner, A., Soc. Neurosci. Abstr. 14:920 (1988); Faissner, A. et al., J. Cell. Biol. 126:783-799 (1994); Schnädelbach, O. et al., Glia 23:99-109 (1998)). In glialen Zellkulturen aus neuralem Gewebe neonataler Ratten wurde durch im m uncytochem ische Analysen die Expression des DSD-1-PG/Phosphacans an bipolaren Vorläuferzellen und größtenteils an ausdifferenzierten Oligodendrozyten detektiert (Garwood, J. et al., Neuroscience 19:51-64 (2001)). Auch bei kultivierten Astrozyten und Oligodendrozyten-Vorläufern wurde die Expression von Phosphacan beobachtet (Dobbertin, A. et al., Mol. Cell. Neurosci. 24:951-971 (2003)).

Interessanterweise ist die Expression der RPTPβ-Isoformen relativ spezifisch für unterschiedliche gliale Zelltypen. So zeigen Oligodendroblasten und Oligodendrozyten ausschließlich eine Expression der langen Isoform des RPTPβ. Hingegen exprimieren ausdifferenzierte Astrozyten überwiegend die kurze Isoform des RPTPβ (Canoll, P. D. et al., J. Neurosci. Res. 44:199-219 (1996); Sakurai, T. et al., J. Neurosci. Res. 43:694-706 (1996)).

Abhängig vom Entwicklungsstadium und der regionalen Spezität der analysierten neuralen Gewebe zeigen alle diese Befunde, dass die Expression des DSD-1-PG/Phosphacans/RPTPβ auf den glialen Vorläufern, den Golgi-Epithelzellen, Astrozyten und Oligodendrozyten lokalisiert und somit gliazellspezifisch ist. Das Ausmaß der Expression des DSD-1-PG/Phosphacans/RPTPβ variiert zwischen den einzelnen Entwicklungsstadien, die durch Zell-Zell- und Zell-ECM-Interaktion bedeutend beeinflusst sind (Maeda, N. et al., Development 122:647-658 (1996); Meyer-Puttlitz, B. et al., J. Comp. Neurobiol. 366:44-54 (1996); Garwood, J. et al., Neuroscience 19:51-64 (2001)). Das DSD-1-PG/Phosphacan, als interaktionsvermitteltendes Molekül, spielt eine besondere regulatorische Rolle in diesen Prozessen, die durch die Interaktion zwischen DSD-1-PG/Phosphacan/RPTPβ und spezifischen Zelladhäsionsmolekülen, wie NCAM, NrCAM, F3Contactin und mehreren anderen Zelloberflächen- und ECM-Molekülen, wie Tenascin-C und Tenascin-R (Milev, P. et al., J. Biol. Chem. 270:24650-24653 (1995); Milev, P. et al., J. Biol. Chem. 273:6998-7005 (1998)) auf positive- oder negative Weise beeinflusst werden (Fawcett, J. W. et al., Brain Res. Bull. 49:377-391 (1999); Bovolenta, P. al., Prog. Neurobiol. 61:113-132 (2000); Hartmann, U. et al., Matrix Biol. 20:23-35 (2001)).

Es sind Antikörper gegen einzelne CSPG bekannt. So offenbaren JP-A-612823/99 und JP-A-631376/95 die Generation von monoklonalen Antikörpern gegen CS-D sowie CS-C unter Verwendung der entsprechenden Strukturen als Antigen. Mit der Bezeichnung CS-C und CS-D werden allerdings zahlreiche Strukturen in diesen Dokumenten zusammengefasst, die erst zum kleinen Teil analysiert sind.

Im Vorfeld der hier vorgestellten Erfindung wurde bereits ein Antikörper gegen eine L2/HNK-1-positive Glykoprotein-Fraktion aus adulten Mäusehirnen vorgestellt (Faissner, A., Neurosci. Abstr. 14:920 (1988)). Dieser Antikörper reagierte mit den Oberflächen unreifer Gliazellen und weiterer, nicht näher charakterisierter neuraler Zellen. Es wurde gezeigt, dass das entsprechende Antigen ein CSPG war. Allerdings blieben die funktionalen Eigenschaften dieses CSPG damals offen.

Überraschenderweise wurde nun gefunden, dass mittels spezieller Agentien (z. B. Antikörper) gegen spezielle Glycosaminglycan-Epitope von Nervenzellgewebe von Vertebraten neurale Stammzellen aus Vertebratengewebe isoliert werden können. Bedingt durch die hohe Konservierung der Struktur der erkannten Glycosaminglycan-Epitope können diese Antikörper auch für die Isolation humaner Stammzellen, insbesondere neuraler Stammzellen verwendet werden, obgleich sie gegen Epitope aus nicht-humanen Quellen erzeugt wurden.

**Zusammenfassung der Erfindung**

Die Erfindung betrifft somit

(1) ein Verfahren zur Isolierung und Kultivierung von neuralen Stammzellen aus Vertebratengewebe, umfassend die Selektion der Stammzellen aus einer Einzelzellsuspension von Vertebratengewebe mittels wenigstens einem gegen DSD-1-Epitope gerichteten Antikörper (primärer Antikörper), der zur spezifischen Bindung mit dem DSD-1-Epitop befähigt ist, wobei das Vertebratengewebe kein humanes embryonales Gewebe ist;

(2) eine bevorzugte Ausführungsform des Verfahrens (1), wobei der primäre Antikörper der monoklonale Antikörper 473HD, der von der Hybridomzelllinie DSM ACC2671 produziert wird, ist;

(3) eine bevorzugte Ausführungsform des Verfahrens (1) oder (2) wobei das Vertebratengewebe in eine Einzelzellkultur überführt wird, vorzugsweise durch Inkubation in Gegenwart einer Protease oder durch mechanische Dissoziation;

(4) eine weitere bevorzugte Ausführungsform des Verfahrens (1) oder (2), wobei die isolierten Stammzellen zur Kultivierung zu Neurosphären verwendet werden;

(5) die Verwendung des in (2) definierten Antikörpers zur Gewinnung von neuralen Stammzellen.

**Kurzbeschreibung der Figuren**

Fig. 1: Struktureller Aufbau des DSD-1-PG/Phosphacan und der RPTPβ Isoformen. Die Carboanhydrase-Domäne (CA) mit den Signalproteinen, die Fibronectin-Domäne (FIII) und Zwischenregion (S) sind bei allen drei Proteoglykanisoformen aufeinander folgend präsent. Die Verbindungsregion (IS) ist bei DSD-1-PG und RPTPβ long vorhanden, nicht aber bei RPTPβ short, die über eine Transmembrandomäne, ähnlich wie RPTPβ long, mit einer intrazellulären Tyrosinphosphatase-Domäne 1 und 2 (PTP 1 und PTP 2) gekoppelt ist.

Fig. 2: Expression des 473HD-Epitopes in telencephalem Cortex während der früheren Embryonalentwicklung. Die immunhistochemische Analyse an frontalen Schnitten des telencephalen Kortex bei E11 (A) zeigt die Lokalisation des 473HD-Epitopes entlang der gesamten ventrikulären Zone. Neben den radial orientierten spezifischen Signalen in der germinalen Zone bei E13 (B) ist die Lokalisation in der sich weiterbildenden kortikalen Platte sehr intensiv. Balken: 50 μm; LV: laterale Ventrikel; VZ: ventrikuläre Zone; CP: kortikale Platte.

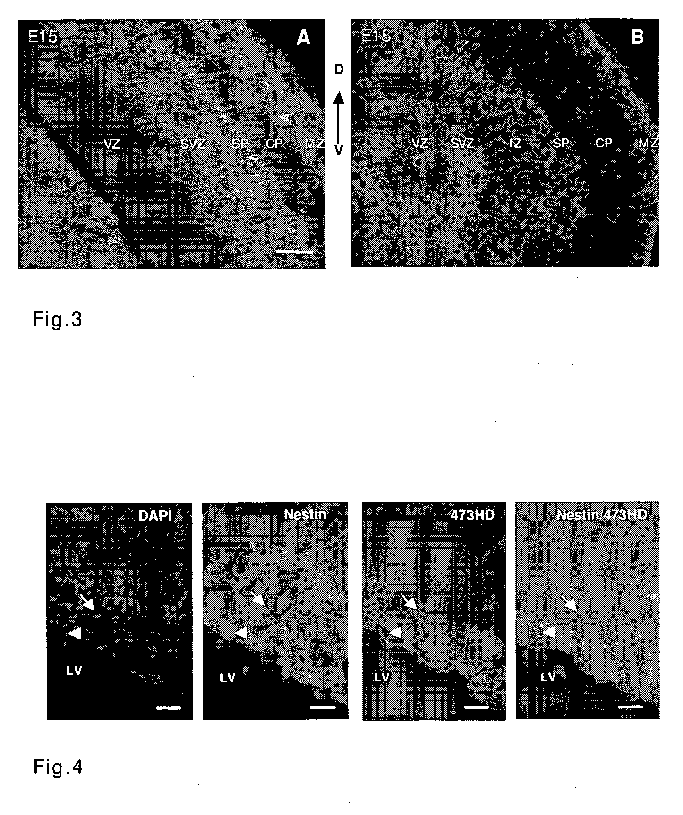

Fig. 3: Expression des 473HD-Epitopes im telencephalen Cortex während der späteren Embryonalentwicklung. Die in späterer embryonaler Entwicklung komplexer werdende Zellschichtarchitektur des telencephalen Kortex ist an frontalen Schnitten des Vorderhirns eines Mausembryos bei E15 (A) und E18 (B) zu erkennen. Die Lokalisation des 473HD-Epitopes ist in primären und sekundären germinalen Regionen sowie in der subkortikalen Platte zu erkennen. Balken: 50 μm; VZ: ventrikuläre Zone; SVZ: subventrikuläre Zone; IZ: intermediäre Zone; SP: subkortikale Platte; CP: kortikale Platte; MZ: Mantelzone.

Fig. 4: Doppelimmunfluoreszenzanalyse mit Antikörper 473HD und Anti-Nestin-Antikörpern an frontalen Schnitten des cerebralen Kortex bei E13. LV: lateraler Ventrikel; Balken: 50 μm.

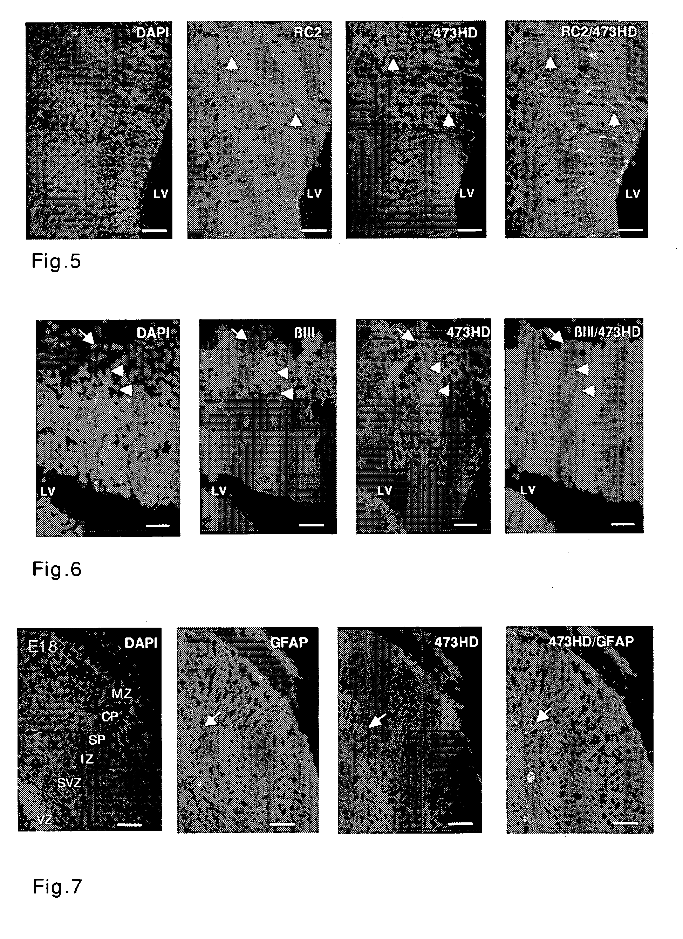

Fig. 5: Doppelimmunfluoreszenzanalyse mit Antikörper 473HD und Anti-RC2-Antikörpern an frontalen Schnitten der lateralen ganglionären Eminenz bei E13 zur Lokalisation des 473HD-Epitopes und der RC2-positiven Zellen. Die Expression des 473HD-Epitopes wurde an einen Teil der radialen Gliazellen in der ventrikulären Zone colokalisiert. Mit Pfeilen sind exemplarisch die Fortsätze einiger radialer Gliazellen markiert, auf denen auch das 473HD-Epitop exprimiert ist. LV: lateraler Ventrikel; Balken: 50 μm.

Fig. 6: Doppelimmunfluoreszenzanalyse mit Antikörper 473HD und Anti-βIII-Tubulin an frontalen Schnitten des cerebralen Kortex bei E13. Die Lokalisation βIIIpositiver postmitotischer Neurone ist in der kortikalen Platte hauptsächlich auf die piale Seite des cerebralen Kortex beschränkt. Eindeutig ist auch die Lokalisation des 473HD-Epitopes in dieser Region. Allerdings weisen die 473HD- spezifischen Signale in der kortikalen Platte keine Co-Lokalisation mit βIII-positiven Zellen auf, sondern befinden sich in der unmittelbaren Umgebung von postmitotischen Neuronen. Die typische radiale Orientierung des 473HD-Epitopes in der ventrikulären Zone ist in der kortikalen Platte nicht vorhanden. LV: lateraler Ventrikel; Balken: 50 μm.

Fig. 7: Die Expression GFAP-positiver Zellen im cerebralen Kortex einer Maus im Embryonalstadium E18. Der DAPI-Anregungsfilter ermöglicht die Darstellung der mit Hoechst markierten Zellkerne. Balken: 50 μm; VZ: ventrikuläre Zone; SVZ: subventrikuläre Zone; IZ: intermediäre Zone; SP: subkortikale Platte; CP: kortikale Platte; MZ: Mantelzone

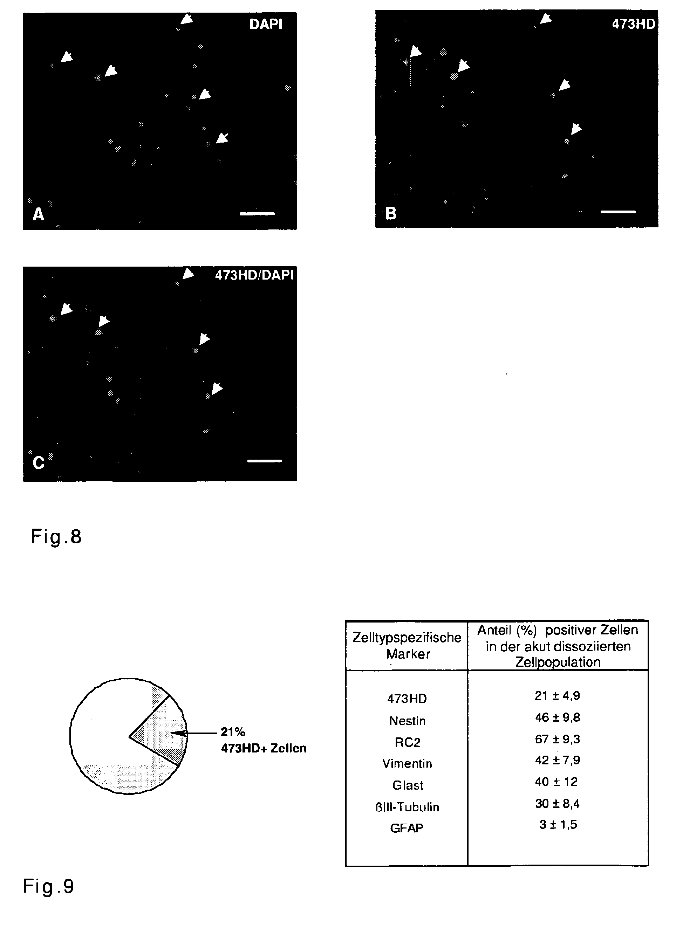

Fig. 8: Expression der membranständigen Isoform DSD-1-PG/RPTPβ an akut dissoziierten Vorderhirnzellen bei E13. Balken: 50 μm.

A: Alle Zellen wurden durch den Kernfarbstoff DAPI (blau) markiert.
B: Indirekte 473HD-Immunfluoreszenzfärbung (CY3, rot) nach 2h *in vitro.*
C: Mehrkanalaufnahme zur Zuordnung des spezifischen 473HD-Signals zu epitopexprimierenden Zellen (Pfeile).

Fig. 9: Zusammensetzung der akut dissoziierten Zellpopulation des Vorderhirns E13

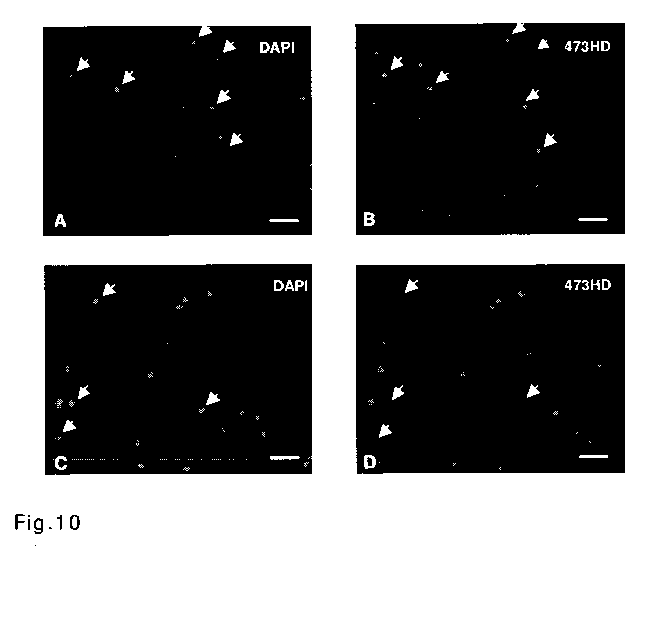

Fig. 10: A und B: Immuncytologische Analyse mit monoklonalem Antikörper 473HD der primären Einzelzellsuspension vor dem Immunopanning. Zellen, die membranständig Isoformen des DSD-1-PG/RPTPβ exprimieren, sind mit weißen Pfeilen markiert.

C und D: 473HD-selektierte Zellpopulation 2h nach dem Immunopanning; nur eine geringe Anzahl Zellen (mit gelben Pfeilen markiert) exprimiert kein 473HD-Epitop. Balken: 50 μm.

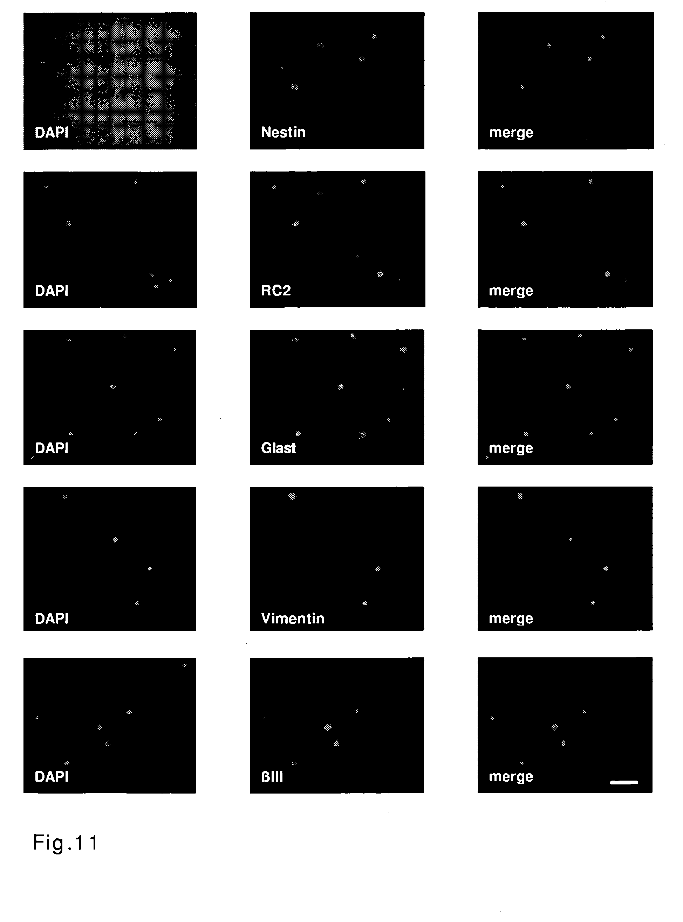

Fig. 11: Fluoreszenzmikroskopische Aufnahmen der immuncytochemischen Färbungen der selektiv isolierten Zellen 2h nach Immunopanning.

Linke Spalte: Kernfärbung (DAPI, blau) aller Zellen
Mittlere Spalte: indirekter Immunfluoreszenznachweis der eingesetzten Marker (CY3, rot bzw. FITC/CY2 grün)
Rechte Spalte: Mehrkanalaufnahmen. Balken: 50 μm

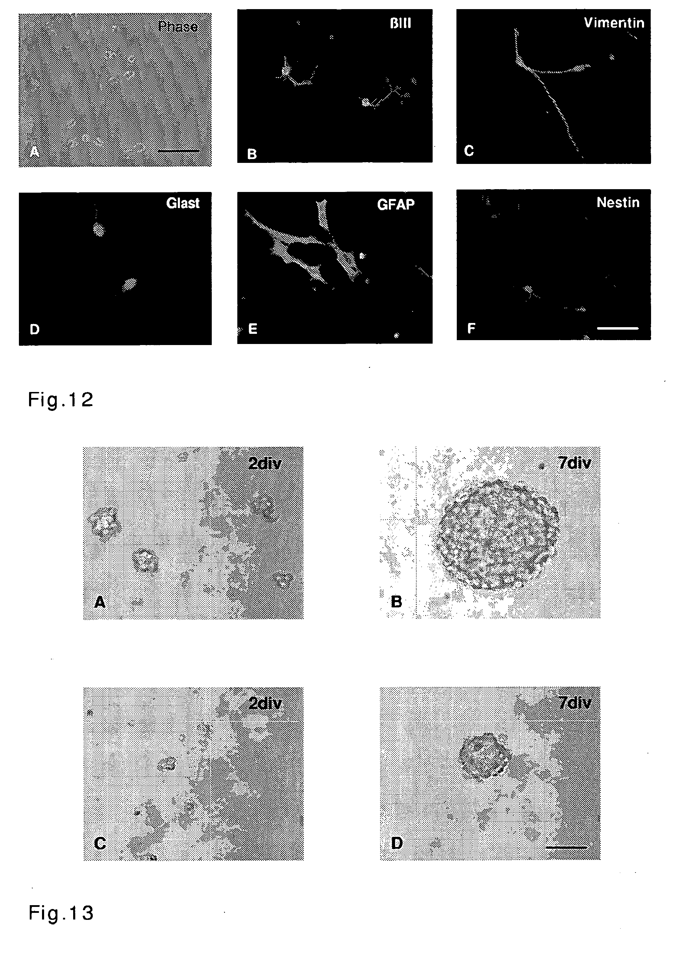

Fig. 12: Fluoreszenzmikroskopische Aufnahmen der immuncytochemischen Färbungen der selektiv isolierten Zellen 2div (Tage *in vitro*) nach Immunopanning. Eine exemplarische Phasenkontrastaufnahme demonstriert einen überwiegenden Anteil von bipolaren Zellen und einige Zellen in Zellteilungsstadien (A). Der größte Teil der Zellen nach 2div war βIII-Tubulin-positiv (B). Neben postmitotischen Neuronen wurden Vimentin-, Glast- und GFAP-positiven Gliazellen (C,D,E) detektiert. Ein geringer Anteil der Zellen zeigte eine Immunreaktivität gegen Nestin-Antikörper (F). Balken: 50 μm.

Fig. 13: Vergleich der Neurosphärenbildung. Neurosphärenbildung aus primär isolierter Einzelzellpopulation (A, B) und aus 473HD-selektierten Zellpopulationen (C, D) unter Standardbedingungen (100.000 Zellen pro 1 ml Neurosphärenmedium) mit EGF und bFGF. Balken: 50 μm.

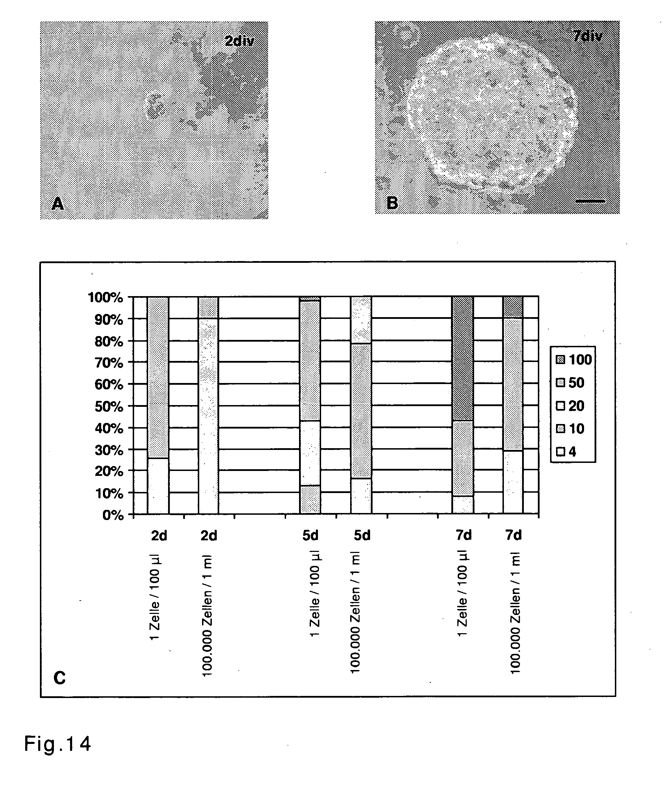

Fig. 14: Vergleich der Neurosphärenbildung aus 473HD-selektierten Zellen bei verschiedenen Zelldichten. Bei Ausplattierung von 1-10 Zellen in 100 μl Neurosphärenmedium pro Napf einer Mikrotiterplatte bilden sich nach 7div größere Neurosphären (A, B) als in gleicher Zeit unter Standardbedingungen mit höherer Plattierungsdichte. Im Diagramm (C) sind die Ergebnisse der Neurosphärenbildung anhand der Kategorisierung der Sphären nach ihrer Größe dargestellt. Die Größen von einzelnen Neurosphären wurden nach 2, 5 und 7div dokumentiert und prozentual (y-Achse) nach Beobachtungstag und Wachstumsbedingungen (x-Achse) zusammengefasst. Balken: 50 μm.

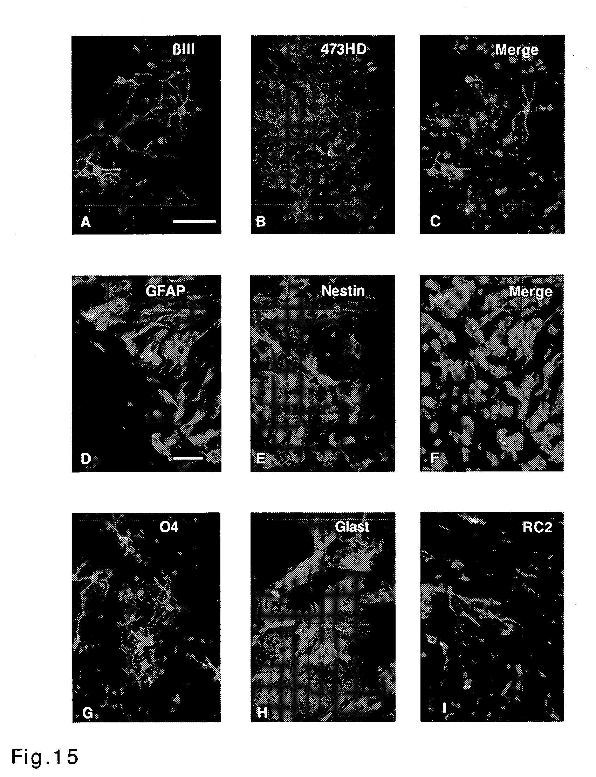

Fig. 15: Immuncytochemische Analyse von primär gebildeten Neurosphären (Anzucht über 7div) aus selektiver Zellpopulation nach 3 Tagen im Differenzierungsassay. Durch Zusatz zelltypspezifischer primärer Antikörper wurden unterschiedliche neurale Zelltypen, wie undifferenzierte Vorläuferzellen (E; Nestin), radiale Gliazellen (I; RC2), gliale Vorläufer (H; Glast), Neurone (A; βIII-Tubulin), Astrozyten (D; GFAP) und Oligodendrozyten (G; 04) an 3d ausdifferenzierten Neurosphären detektiert. Mit Antikörper 473HD wurden DSD-1-PG/Phosphacan-exprimierende Zellen detektiert (B). Balken: 70 μm in A, B, C, G; 50 μm in D, E, F, H, I.

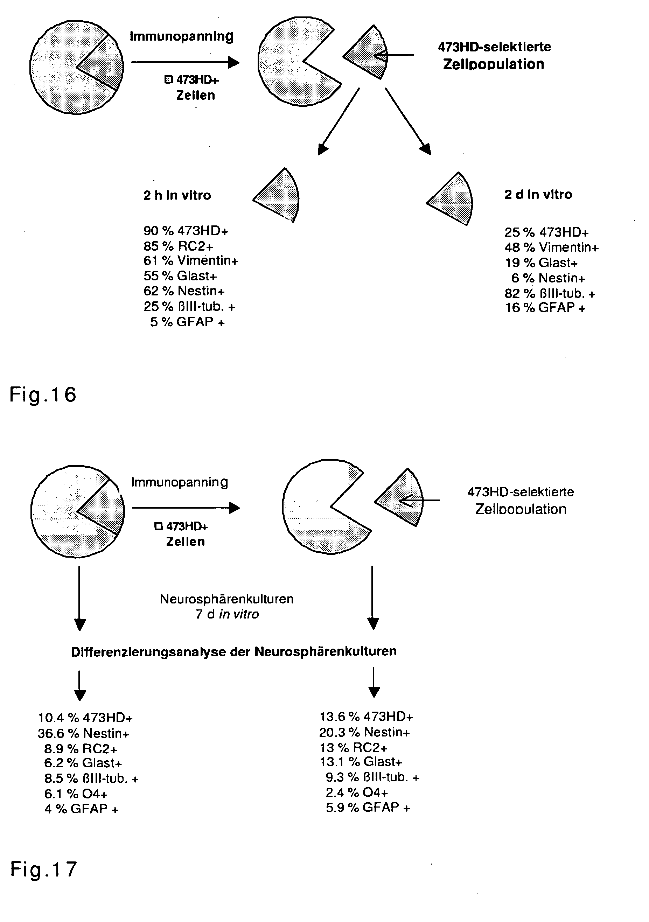

Fig. 16: Ergebnisse der immuncytochemischen Analyse einer 473HD-selektierten Zellpopulation nach 2h und 2d *in vitro*

Fig. 17: Vergleich der immuncytochemischen Analyse von ausdifferenzierten primären Neurosphären nach 7 d *in vitro* aus primärer Einzelzellsuspension (links) vs. aus 473HD-selektierter Zellpopulation (rechts).

Detaillierte Beschreibung der Erfindung

"Vertebraten" im Sinne der vorliegenden Erfindung umfassen Säuger, insbesondere Mensch und Nager, bei den Nagern ganz besonders Maus oder Ratte, sowie Vögel, Amphibien und Fische. Weiterhin kann das verwendete Vertebratengewebe nicht-humaner embryonaler, postnataler oder adulter Herkunft sein.

Bei dem erfindungsgemäßen Verfahren gemäß Ausführungsformen (1) oder (3) wird eine Einzelzellsuspension aus embryonalem, postnatalem und/oder adultem Gewebe, insbesondere aus Nervengewebe oder Gehirn hergestellt.

"Stammzellen" im Sinne der vorliegenden Erfindung sind teilungsfähige Zellen des nicht-humanen Embryos, des Fötus oder des Erwachsenen, die noch nicht vollständig differenziert sind und somit entweder identische Zellen oder verschiedene differenzierte Zelltypen bilden können. Stammzellen können toti- oder pluripotente Stammzellen ektodermalen oder neuroektodermalen Ursprungs (z. B. neuronale Stammzellen), mesodermalen Ursprungs (z. B. hematopoetische und mesenchymale Stammzellen), endodermale Stammzellen (z.B. gastrointestinale Stammzellen), Stammzellen der Neuralleiste (z. B. Stammzellen des peripheren Nervensystems) sein. "Stammzellen" umfassen somit nicht-humane embryonale Stammzellen (ES-Zellen), nicht-humane primordiale Keimzellen (EG-Zellen), nicht-humane embryonale Krebszellen. (EC-Zellen) oder adulte (somatische) Stammzellen (AS-Zellen). Stammzellen können hämatopoetische Stammzellen, Amnionzellen, mesenchymale Stammzellen, neurale Stammzellen, endotheliale Stammzellen, gastrointestinale Stammzellen, epidermale Stammzellen, Hirn-Stammzellen, Muskel-Stammzellen usw. sein.

Mit dem Verfahren gemäß Ausführungsformen (1) und (3) sowie (5) der Erfindung können neuronale Stammzellen isoliert werden.

Im folgenden wird der Gegenstand der vorliegenden Anmeldung näher beschrieben. Das Ergebnis des Beispiels 9 legt jedoch nahe, dass auch andere Zellen, die das 473HD-Epitop an der Oberfläche exprimieren, mit Hilfe des monoklonalen Antikörpers 473 HD immunsortiert werden können.

Obgleich die experimentellen Beispiele im Wesentlichen nur murine Stammzellen betreffen, können mit der erfindungsgemäßen Methode auch Stammzellen anderer Säugetiere, insbesondere Stammzellen des Menschen isoliert werden. Es gibt viele Beispiele dafür, dass komplexe Kohlenhydrate als Marker in der Zell- und Humanbiologie konserviert sind. Beispiele sind das Sulfatid-Antigen 04 als Marker für frühe Oligodendrozyten in der Maus und im Menschen, sowie die Lewis-X Blutgruppenantigene.

Im Hinblick auf die Frage, ob das DSD-1-Epitop auch in menschlichen Geweben nachweisbar ist, wurde eine systematische Analyse diverser Zelllinien vorgenommen. Die Untersuchungen wurden auf die Zelllinien S117 und WISH sowie auf verschiedene humane Gliomazelllinien, z.B. U-87MG, konzentriert. S117 dient als Modell für Sarkome des Menschen, malignen Tumoren des Bindegewebes und Knochens (siehe z. B. Roszinski, S. et al., J. Radiat. Oncol. Biol. Phys. 20:1273-1280 (1991)). Die WISH Zellen sind transformierte humane Amnionepithelzellen, die Bindegewebe in Wirtsgeweben induzieren können (Wlodarski, K. et al., Calcif. Tissue Res. 5:70-79 (1970)). Die WISH Zelllinie repräsentiert ein etabliertes Modell, das - ebenso wie die S117-Linie - in der biomedizinischen Forschung seit mehreren Jahrzehnten kontinuierlich eingesetzt wird (Fiorini, S. et al., Endocrinology 144:3359-3367 (2003)). Das Mab 473 HD-Epitop konnte bei beiden Zelllinien immunzytologisch in den Zellmembranen und auf dem Zellkultursubstrat nachgewiesen werden (zu WISH vgl. Bsp. 9). Eine intensive Substratkonditionierung konnte auch mit der humanen Glioma-Zelllinie U-87MG (Ponten, J. und Macintyre, E.H., Acta Path. Microbiol. Scandinav. 74:465-486 (1968)) nachgewiesen werden (Bsp. 9; Wendel, C., "Immuncytologische, immunchemische und funktionelle Untersuchungen zu RPTP-zeta/beta Expression in humanen Tumorzellen", Diplomarbeit, Fakultät für Biologie der Ruhr-Universität Bochum (2005)). Die Substratkonditionierung indiziert eine Freisetzung des Epitops in das Medium. Mab 473HD-positives, polydisperses, Chondroitinase ABC-sensitives Material konnte im Kulturüberstand von radioaktiv mit $^{35}SO_4$-markierten WISH-Zellen dokumentiert werden. Damit ist nachgewiesen, dass das DSD-1-Epitop in menschlichen Zelllinien - und in entsprechenden menschlichen Geweben - mit hoher Wahrscheinlichkeit in der Form eines Proteoglykans auftritt (A. Faissner, unveröffentlichte Beobachtungen) .

Beispiel 9 zeigt anhand der Ergebnisse für WISH und U-87MG, dass auch humane Zellen das 473HD-Epitop an der Oberfläche exprimieren. Im Lichte dieses Beispiels kann davon ausgegangen werden, dass ebenfalls das spezifische, von Mab 473HD identifizierte Chondroitinsulfat-Antigen in der Humanbiologie eine analoge Rolle zu der in der Maus nachgewiesenen spielt.

In einer bevorzugten Ausführungsform von (1) weist das primäre Agenz wenigstens eine Bindungsstelle für ein spezifisches sekundäres Agenz auf. Solche Bindungsstellen können Epitope für Antikörper oder auch an das primäre Agenz gekoppelte Teile eines Immunbindungspaares sein. Als primäres Agenz können insbesondere gegen DSD-1-Epitope gerichtete monoklonale oder polyklonale Antikörper (primärer Antikörper) eingesetzt werden. Besonders bevorzugt sind dabei monoklonale Antikörper. Weitere primäre Agenzien sind spezifische Glycosaminoglykan-bindende Proteine oder daraus abgeleitete funktionelle Domänen oder rekombinant hergestellte Proteine. Zu letzteren zählen insbesondere Expressionskonstrukte aus "phage display libraries" oder auf einem analogen Prinzip beruhende Proteine, die spezifisch mit dem DSD-1-Epitop, mit Chondroitinsulfatglykosaminoglykanen oder verwandten Kohlenhydraten reagieren. Sekundäre Agenzien umfassen ebenfalls Antikörper (sekundäre Antikörper), Teile von Immunbindungspaaren (z.B. von Biotin/Streptavidin u.s.w.). Die sekundären Antikörper können mit funktionalen Verbindungen, wie z.B. den vorstehend genannten Teilen eines Immunbindungspaares, Enzymen, magnetic beads, etc. versehen sein.

In einer bevorzugten Ausführungsform von (1) ist das primäre Agenz ein Antikörper (primärer Antikörper), der besonders bevorzugt ein Epitop für einen sekundären Antikörper aufweist. Als primärer Antikörper können humane, murine, Ratten-, Kaninchen-, Ziegen- oder Schweineantikörper eingesetzt werden. Primäre Antikörper sind bevorzugt entweder durch Immunisieren von Ratten mit einer Präparation, umfassend Glykoproteine, Proteoglycane und Glycoproteinfraktionen adulter Mäusehirnmembrane, erhältlich, und/oder weisen Epitope für einen anti-Ratte Antikörper auf, welcher bevorzugt die Subklassen IgG, IgA, IgE, IgM oder die leichten Ketten χ und λ erkennt. Besonders bevorzugt als primärer Antikörper im Verfahren gemäß (1) ist der monoklonale Antikörper 473HD, welcher von der Hybridomzelllinie DSM ACC2671 produziert wird.

Besonders bevorzugt als sekundärer Antikörper sind anti-Ratte Ig CY3 oder anti-Ratte Ig biot+ Streptavidin FITC.

In einer weiteren bevorzugten Ausführungsform ist das primäre Agenz durch geeignete Methoden zur Immobilisierung an ein Trägermaterial gekoppelt. Derartige geeignete Immobilisierungsmethoden umfassen adäquate Kupplungstechniken, welche die Spezifität des primären Agenz nicht verändern, wie z.B. die kovalente Vernetzung des primären Antikörpers mit dem Trägermaterial. Bevorzugt erfolgt die Immobilisierung des primären Antikörpers an ein Trägermaterial durch Wechselwirkung mit dem erfindungsgemäßen sekundären Antikörper.

Ebenfalls ist bevorzugt, dass durch spezifische Bindung neuraler Stammzellen aus einer Einzelzellsuspension an das immobilisierte oder nicht immobilisierte primäre Agenz eine Isolierung dieser Zellen aus der Zellsuspension erfolgt, die nicht gebundene Zellen entfernt werden und anschließend die über das primäre Agenz und gegebenenfalls den sekundären Antikörper an das Trägermaterial gebundenen Zellen durch Inkubation mit einem geeigneten Reagenz, besonders bevorzugt einer geeigneten Protease, vom Trägermaterial abgelöst werden. Das Verfahren erfolgt vorzugsweise *in vitro*. Die auf diese Weise isolierten Zellen können als Ausgangspunkt für weitere Zellkulturen, besonders für Einzelzellsuspensionen oder für die Herstellung von Neurosphären, zur immuncytochemischen Untersuchung, zur Herstellung von Hybridomzellen, zur Anzucht von differenzierten Nervenzellen für Therapiezwecke und zum Einsatz in weiteren cytochemischen Experimenten verwendet werden.

Gemäß Ausführungsform (3) der Erfindung wird das Vertebratengewebe durch Inkubation in Gegenwart einer Protease in eine Einzelzellkultur überführt. Bevorzugte Proteasen sind dabei Serin-Proteasen, wobei Trypsin besonders bevorzugt ist. Bevorzugt ist des weiteren, dass sich an die Protease-Behandlung eine Erholungsphase anschliesst. Diese Erholungsphase richtet sich besonders bevorzugt nach dem Kriterium der optimalen Vitalität der Zellen. Für die Länge der Erholungsphase ist zusätzlich oder alternativ besonders bevorzugt, dass die Zellen der Einzelzellkultur nach Abschluss der Protease-Behandlung vor Beginn der Selektion 0,5-6 h, bevorzugt 1-4 h, besonders bevorzugt 1-2 h und ganz besonders bevorzugt etwa 2 h in einem geeigneten Medium oder Puffer inkubiert werden.

Die isolierten neuralen Stammzellen können gemäß Ausführungsform (4) zu Neurosphären kultiviert werden. Die Kultivierung erfolgt in hierfür geeigneten Kulturmedien unter Standardbedingungen, bevorzugt in serumfreiem Medium, besonders bevorzugt in einem Neurosphärenmedium enthaltend Dulbecco's Modified Eagle's Medium (DMEM) (Sigma: D-6546) und F12 (Sigma: N-4888) im Verhältnis 1:1, plus L-Glutamin (Sigma: G-5763) 0,2 mg/ml, B27 (Gibco: 17504-044) 2 ml/100ml, Penicillin/Streptomycin (Gibco: 15140-122) 10 µl/ml.

Die vorliegende Erfindung beruht auf dem überraschenden experimentellen Befund, dass eine das DSD-1-Epitop exprimierende Zellpopulation selektiv isoliert und immuncytochemisch *in vitro* charakterisiert werden kann. Die Isolation basiert auf der Spezifität von speziellen Agenzien, wie der des monoklonalen 473HD Antikörpers für das CSD-abhängige DSD-1-Epitop. Insbesondere können so Zellen isoliert werden, die Phosphacan, DSD-1-PG, RPTP-β/ζ (long) exprimieren.

Die Ergebnisse unterschiedlicher zellbiologischer Methoden zeigten, dass es sich bei den 473HD-Epitop exprimierenden Zellen aus dem Vorderhirn des Stadiums E13 hauptsächlich um radiale Gliazellen handelt. Die 473HD-positive Zellpopulation zeigte unter verschiedenen Kulturbedingungen *in vitro* eine Reihe spezifischer Eigenschaften, die sowohl für radiale Gliazellen als auch für multipotente neuroepitheliale Stammzellen charakteristisch sind. Die vorliegende Erfindung ermöglichte damit den Nachweis, dass DSD-1-PG/Phosphacan/RPTPβ während der embryonalen Entwicklung in der proliferativen Region des telencephalen Kortex exprimiert wird. Bei der immunhistochemischen Analyse der Expression des 473HD-Epitops im Vorderhirn von Mäusen *in vivo* im Embryonalstadium E11, E13, E15 und E18 wurde folgendes gefunden (vergleiche auch Beispiel 6):

Bei E11, also schon vor Beginn der Neurogenese war die Expression des CS-D-abhängigen Epitops von DSD-1-PG/Phosphacan/RPTPβ in der gesamten ventrikulären Zone des telencephalen Kortex und der ganglionären Eminenz nachweisbar. Die Expression wies eine radiale Orientierung zwischen der ventrikulären und der pialen Oberfläche auf und konnte zum Teil an einzelnen neuroepithelialen Zellfortsätzen beobachtet werden (Fig. 2A). Die neuroepithelialen Zellen in der ventrikulären Zone aller Vorderhirnregionen wurden bei immunhistochemischen Analysen an Vorderhirnschnitten von Mäuseembryonen ab E11 durch Nestin markiert. Somit kann vermutet werden, dass die neuroepithelialen Zellen das 473HD-Epitop exprimieren. Allerdings lassen sich schon kurz vor Beginn der Neurogenese zwei morphologisch unterschiedliche Subtypen neuroepithelialer Zellen in der ventrikulären Zone unterscheiden: Eine große Anzahl dieser Zellen verliert ihren Kontakt zur pialen Oberfläche, und befindet sich in den proliferativen Schichten des Vorderhirns. Die anderen Zellen kontaktieren mit ihren Zellfortsätzen sowohl die ventrikuläre als auch die piale Oberfläche (Huttner, W. B., Brand, M., Neurobiol. 7:29-39 (1997); Malatesta, P. et al., Development 127:5253-5263 (2000)). Letztere Zellen werden als radiale Gliazellen bezeichnet, die sich durch ein spezifisches Antigenprofil von anderen neuroepithelialen Zellen unterscheiden lassen (Hartfuss, E. et al., Dev. Biol. 229:15-30 (2001)) und eine entscheidende Funktion für die Koordinierung der Neuronenmigration haben (Rakic, P., Science 183:425-427 (1972); Hatten; M.E. et al., Annu. Rev. Neurosci. 22:511-539 (1999)). Somit kann nicht ausgeschlossen werden, dass bereits bei E11 das 473HD-Epitop auf radialen Gliazellen exprimiert ist.

Bei E13 wurde die Lokalisation des 473HD-Epitopes in der ventrikulären Zone des gesamten telencephalen Kortex nachgewiesen und zeigte hier eine radiale Orientierung zwischen ventrikulärer und pialer Oberfläche (Fig. 2B). Eine kompaktere und eher tangential orientierte Expression wurde in der sich bildenden subventrikulären Zone der ganglionären Eminenz (GE) beobachtet. Dies hängt möglicherweise mit Besonderheiten der Migration einiger Neuronen zusammen, da kortikale Interneurone durch tangentiale Migration aus der GE zu ihrer endgültigen Lokalisation im Kortex gelangen (Anderson, S. A. et al., Science 278:474-476 (1997); Pearlman, T. et al., Genes Dev. 9:769-782 (1998); Tan, S. S. et al., Neuron. 21:295-304 (1998)). Da radiale Gliazellen die Neuronenmigration koordinieren und entlang der Migrationsbahnen lokalisiert sind, läßt sich vermuten, dass sowohl in der neokortikalen als auch in der striatalen Region des sich entwickelnden Telencephalons die radialen Gliazellen eine Quelle des 473HD-Expressionsmusters darstellen. Durch eine Doppelimmunfluoreszenzanalyse unter Anwendung der monoklonalen RC2- und 473HD-Antikörper wurde diese Annahme bestätigt, weil die Expression des 473HD-Epitopes zum großen Teil an RC2-immunreaktiven radialen Gliazellen lokalisiert wurde (Fig. 5). Die Orientierung des 473HD-Expressionsmusters stimmte mit der Lokalisation der RC2-positiven Zellen im gesamten Vorderhirn bei E13 überein.

Die weitere Entwicklung des telencephalen Kortex ist durch das Entstehen einer immer komplexer werdenden Zellschichtarchitektur gekennzeichnet und weist bei E15 - zu Beginn der Gliogenese - neben der primären auch die sekundäre germinale Zone (subventrikuläre Zone) auf. Hier findet hauptsächlich die Proliferation der glialen Vorläufer statt (Davis, A. A., Temple, S., Nature 372:263-266 (1994); Mayer-Proschel, M. et al., Neuron. 15:773-785 (1997); Mujataba, T. et al., Dev. Biol. 214:113-127 (1999)). Auch in dieser Region aktiver Zellproliferation wurde eine hohe Expression des 473HD-Epitopes nachgewiesen (Fig. 3), welches hier fast ausschließlich entlang der radial orientierten Zellfortsätze detektiert wird. In der kortikalen Platte zeigt sich wiederum eine radiale Orientierung und in der marginalen Zone eine eher tangential ausgerichtete Expression (Fig. 3A).

Die Expression des 473HD-Epitopes wurde auch bei E18 in der sekundären proliferativen Zone lokalisiert (Fig. 3B). Bei E18 wurde schliesslich im Gegensatz zu E11, E13 und E15 die Expression von 473HD an der Zellmembran von GFAP-positiven Zellen co-lokalisiert (Fig. 7). In diesem relativ späten Entwicklungsstadium ist die Neuronenmigration abgeschlossen und die radialen Gliazellen transformieren zu Astrozyten (Voigt, T., J. of Comparative Neurobiol. 289:74-88 (1989); Edwards, M. A. et al., Neurosci 36:12-144 (1990); Culican, S. M. et al., J. Neurosci. 10:684-692 (1990)). Daher ist es nicht auszuschließen, dass es sich bei der nachgewiesenen Expression des 473HD-Epitopes um Expression auf Astrozyten handelt.

Insgesamt zeigten die immunhistochemischen Analysen in unterschiedlichen Embryonalstadien eine Expression von DSD-1-PG/Phosphacan/RPTPβ, die außer in der germinalen Zone auch in der marginalen Zone sowie der kortikalen Platte lokalisiert ist und ein zellschichttypisches und regionspezifisches Muster aufweist. Des Weiteren konnte festgestellt werden, dass die Veränderung der Lokalisation des 473HD-Epitopes bei allen untersuchten embryonalen Stadien mit den stattfindenden Entwicklungsprozessen, wie Proliferation der neuralen Stammzellen, Zellmigration und Differenzierung, sowie Formation der telencephalen Strukturen, korrespondiert. Die Expression des 473HD-Epitopes wurde in allen untersuchten Embryonalstadien in ventrikulärer und subventrikulärer Zone, Regionen aktiver Zellproliferation, entlang der radial orientierten Zellfortsätzen und in der marginaler Zone gefunden. Dies lässt den Schluss zu, dass radiale Gliazellen das 473HD-Epitop tragen.

Die Ergebnisse der immunhistochemischen Analysen an Vorderhirnschnitten erlaubten zwar die qualitative Aussage über die Lokalisation und das Expressionsmuster des 473HD-Epitops bezüglich kortikaler und striataler Zellschichtarchitektur im embryonalen ZNS, ermöglichten aber keinerlei Aussagen über die Größe der epitopexprimierenden Zellpopulation oder eine eindeutige Identifikation des Zelltyps oder der Zelltypen, die in dieser Entwicklungsperiode das 473HD-Epitop exprimieren.

Die immuncytochemischen Analysen an akut dissoziierten Zellen aus dem Vorderhirn (E13) ergaben, dass 21 $\pm$ 4,9% der Zellen das 473HD-Epitop exprimieren (Fig. 8, Bsp. 7A). Neben der 473HD-Expression wies die Einzelzellpopulation βIII-positive post-mitotische Neurone und Nestin-positive Vorläuferzellen auf. Ein großer Anteil der Zellen exprimierte für radiale Gliazellen spezifische Epitope: 67 $\pm$ 9,3% der Zellen zeigte eine Immunreaktivität gegen RC2-Antikörper, sowie gegen Glast- und Vimentin-Antikörper (Fig. 9), während nur 21 $\pm$ 4,9% der akut dissoziierten Zellen das 473HD-Epitop exprimierten. Allerdings ist bekannt, dass die Population der radialen Gliazellen Subtypen aufweist, die sich durch molekulare und morphologische Charakteristika unterscheiden (Hartfuss, E. et al., Developmental Biol. 229:15-30 (2001)). Es war davon auszugehen, dass nicht alle Subtypen der radialen Gliazellen das DSD-1-PG/RPTPβ-long exprimieren.

Um die 473HD-exprimierenden Zellen zu charakterisieren, wurde diese Zellpopulation durch Immunopanning selektiv angereichert (Beispiel 7B). Die Spezifität des monoklonalen 473HD-Antikörpers ermöglichte die selektive Isolierung der DSD-1-PG/RPTPβ-exprimierenden Zellen. Voraussetzung für ein erfolgreiches Immunopanning ist das Vorhandensein eines auf der extrazellulären Seite der Zelle exprimierten, membranständigen Epitopes, das durch Wechselwirkung mit spezifischen Antikörpern einen Antigen-Antikörper-Komplex bilden kann. Für das 473HD-Epitop, welches an den Glykosaminoglykanketten der DSD-1-PG/RPTPβlong exprimiert wird (Faissner, A. et al., J. Cell. Biol. 126:783-799 (1994); Garwood, J. et al., J. Neurosci. 19:3888-3899 (1999)), trifft dies zu.

Durch Immunopanning nach enzymatischer Dissoziation des Gewebeverbandes wurden 57% der 473HD-positiven Zellen aus der Gesamtpopulation isoliert (vgl. Bsp. 7B). Die Isolierung der Hälfte der 473HD-Epitop exprimierenden Zellen ist für Immunopanning ein sehr gutes Ergebnis, da schon während der enzymatischen Gewebedissoziation Veränderungen der Zellmembran auftreten, durch die das 473HD-Epitop an der Zelloberfläche verloren geht. Ein weiterer Verlust von Zellen durch weitere Verfahrensschritte wie z.B. Zentrifugation erklärt, warum eine höhere Ausbeute kaum erreicht werden kann, was auch einige etablierte Immunopanningprotokolle für andere Zelltypen konstatieren (Barres, B. A. et al., Cell 70:31-46 (1992); Barres, B.A. et al., J. Cell Biol. 147:1123-1128 (1999)).

Zu diesen technischen Aspekten kommt hinzu, dass nicht alle Zellen in der primären Zellsuspension, die das DSD-1- PG/ RPTPβ aufweisen, das 473HD-Epitop in gleicher Menge exprimieren. Wahrscheinlich führt nur eine starke Expression der membranständigen Isoform DSD-1-PG/RPTPβ zur selektiven Zellisolierung durch das Immunopanning.

Die Qualität selektiver Isolierungsmethoden wird hauptsächlich durch den Anreicherungswert bestimmt. Die maximale Anreicherung betrug bei den durchgeführten Immunopanning-Experimenten 98,7%. Dies kann in erste Linie dadurch begründet werden, dass nach der zweifachen enzymatischen Gewebedissoziation die Inkubationszeit von 2 h nach dem Immunopanning nicht ausreicht, um die 473HD-Expression an der Zelloberfläche wieder in allen Zellen so hoch zu regulieren, dass diese bei der quantitativen Bestimmung durch Immunfluoreszenz vollständig erfasst werden. Eine Verlängerung der Inkubationszeit nach dem Immunopanning kann folglich zu einer höheren Anreicherung der 473HD-positiven Zellen führen.

Alternativ kann durch zusätzliche Waschschritte vor der Trypsinierung der Panningschale eine Verringerung der Kontamination mit 473HD-negativen Zellen erreicht werden, wobei gleichzeitig mit dem weiteren Verlust von 473HD-positiven Zellen gerechnet werden muss.

Letztendlich könnte eine höhere Effizienz auch über sequentielle Selektion erreicht werden. Dabei wird die positiv isolierte Zellpopulation erneut selektioniert und somit weiter angereichert. Alle genannten Möglichkeiten zur Steigerung der Anreicherung der 473HD-positiven Zellpopulation sollten für die weitere Optimierung des Immunopanningprotokolls in Betracht gezogen werden.

Insgesamt ermöglichte die spezifische Bindung des monoklonalen 473HD-Antikörpers an die CS GAG-Ketten die selektive Isolierung der DSD-1-Epitop exprimierenden Zellen durch das Immunopanning. Dies stellt eine sehr gute Ausgangsbasis für die Charakterisierung der 473HD-positiven Zellpopulation dar. Da in Maus nur zwei Isoformen des Phosphacans, die sekretierte Isoform DSD-1-PG und die RPTPβ long Isoform, die CS GAG-Ketten aufweisen, handelt es sich bei den durch das Immunopanning isolierten Zellen mit größter Wahrscheinlichkeit um Träger der RPTPβ-long-Isoform.

Das 473HD-Epitop ist auf einem Subtyp der radialen Gliazellen exprimiert. Die immuncytochemische Analyse der 473HD-selektierten Zellen 2 h nach der Isolierung zeigte, dass 62% dieser Zellen Nestin-positiv und 85% der Zellen RC2-positiv sind (Fig. 12, Fig. 16, Beispiel 8A). Es ist bereits bekannt, dass bis zu 98% der RC2-positiven Zellen auch Nestin-positiv sind (Malatesta, P. et al., Development 127:5253-5263 (2000)). Mehr als die Hälfte der isolierten Zellen zeigen Immunreaktivität für weitere radiale Gliamarker, z.B. Glast.

Die Subtypen der radialen Gliazellen lassen sich unabhängig von morphologischen Charakteristika eindeutig nur durch spezifische immunologische Profile unterscheiden. Dabei wird zwischen RC2+, RC2+/Glast+, RC2+/BLBP+ und RC2+/Glast+/BLBP+ Subtypen radialer Gliazellen unterschieden, die entwicklungsphasenspezifisch auftreten (Hartfuss, E. et al., Developmental Biol. 229:15-30 (2001)). So werden im cerebralen Kortex während der Neurogenese (E12-17) neben dem geringen Anteil (12%) der RC2+ Zellen bis zu 42% RC2+/Glast+/BLBP+ und 34% RC2+/Glast+ detektiert. Zu Beginn der Gliogenese weist das kortikale Gewebe aber nur zwei Subtypen radialer Gliazellen (67% RC2+/ Glast+/BLBP+ und 33% Glast+/BLBP+) auf (Hartfuss, E. et al., Developmental Biol. 229:15-30 (2001)).

Neben oben beschriebenen immunpositiven Zellen wies die 473HD-selektierte Zellpopulation zu einem Viertel βIII-Tubulin-positive Zellen auf (Fig. 16). Der Anteil βIII-Tubulin-positiver Zellen lag bei allen durchgeführten Experimenten in dieser Größenordnung, und zwar unabhängig vom Anreicherungswert in den einzelnen Experimenten. Wahrscheinlich ist, dass die teilungsaktiven radialen Gliazellen in der Zeit zwischen dem Immunopanning und der immuncytochemischen Analyse der selektiv isolierten Zellpopulation sich asymmetrisch teilen und in diesem Zeitraum beginnen, sich zu Neuronen zu differenzieren. Möglicherweise zeigen diese Neuroblasten nicht nur Immunreaktivität gegen βIII-Tubulin, sondern tragen auch noch während der Differenzierung in geringer Expression radiale Gliazellmarker. Dadurch lässt sich erklären, dass eine geringe Zellpopulation eine Kombination aus RC2, βIII-Tubulin und Nestin-Epitopen gleichzeitig exprimiert, wie auch von anderen Gruppen beschrieben (Hartfuss, E. et al., Soc. Neurosci. Meet. 25:522 (1999); Noctor et al., Nature 409:714-720 (2001)).

Die nach 2 div durchgeführten immuncytochemischen Analysen ermöglichten die Bestimmung der Veränderung in der Zusammensetzung der selektierten Zellpopulation. Dabei wurde gezeigt, dass die überwiegende Anzahl der 473HD-positiven Zellen *in vitro* zu Neuronen (βIII-Tubulin-positiven Zellen) differenzieren, die nach 2 div 82% der gesamten Zellpopulation ausmachen (Fig. 12). Die Expression des 473HD-Epitopes sowie die Nestin-, Vimentin- und Glastimmunreaktivität gingen gleichzeitig zurück. Dieses Ergebnis bestätigt weiterhin die Annahme, dass die radialen Gliazellen nicht nur eine Funktion bei der Koordination der Neuronenmigration während der Neurogenese übernehmen, sondern aktiv an diesem Entwicklungsprozess teilnehmen. Dies korrespondiert mit Beobachtungen und Ergebnissen anderer Arbeitsgruppen (Malatesta, P. et al., Development 127:5253-5263 (2000); Malatesta, P. et al., Neuron 37: 751-764 (2003); Miyata, T. et al., Neuron 31:727-741 (2001); Hartfuss, E. et al., Developmental Biol. 229:15-30 (2001); Noctor et al., Nature 409:714-720 (2001); Tamamaki, N. et al., J. Neurosci. Res. 41:51-60 (2001)).

Einige 473HD-positive Zellen zeigen Charakteristika der neuralen Stammzellen. Aus zellbiologischer Sicht ist das embryonale Stadium E13 besonders interessant, da kurz vor diesem Zeitpunkt die Expansionsphase der neuralen Stammzellen endet und die beginnende Neurogenese zur Entstehung erster neuronaler Vorläufer führt (Temple, S., Nature 414:112-117 (2001)). Die immunhistologische Analyse an Hirnschnitten bei E13 *in vivo* ergab jedoch keine Co-Lokalisierung des 473HD-Epitopes an βIII-Tubulin-positiven Zellen (Fig. 6). Daher kommen nur die Stammzellen und/ oder radialen Gliazellen, die sich in proliferativen Regionen des Vorderhirns befinden, als DSD-1-PG/RPTPβ-exprimierende Zellen in Frage.

Neurale Stammzellen sind durch ihre auch *in vitro* beibehaltenen Eigenschaften wie Proliferationsaktivität und Pluripotenz charakterisiert. Dies äußert sich in der Bildung von Zellkolonien, den Neurosphären, die aus je einer Stammzelle in serumfreiem Medium nach Zugabe von Wachstumsfaktoren entstehen. Die Wachstumsfaktoren bFGF (basic Fibroblast Growth Factor) und EGF (Epidermal Growth Factor), die während der embryonalen Entwicklung im ZNS vorkommen, sind *in vitro* für die Neurosphärenbildung in serumfreiem Medium essentiell.

18 ± 4% der selektiv isolierten 473HD-positiven Zellen zeigten das Potential zur Neurosphärenbildung (Beispiel 8B). Die Neurosphärenbildung verlief jedoch im Vergleich zu Zellen aus einer primär isolierten Einzelzellsuspension wesentlich langsamer (Fig. 13). Des weiteren wurde eine deutliche Beschleunigung der Neurosphärenbildung bei geringerer Dichte der selektiv isolierten Zellen festgestellt. Diese Beobachtung korreliert mit den Ergebnissen einer systematischen Studie, in welcher der Zusammenhang zwischen ausplattierter Zelldichte und der Anzahl gebildeter Neurosphären gezeigt wurde (Tropepe et al., Dev. Biol. 208:166-188 (1999)).

Somit stand fest, dass ein Teil der 473HD-positiven Zellen Stammzell-charakteristika aufweist und in der Lage ist, Proliferationsaktivität *in vitro* beizubehalten. Dass über 80% der 473HD-selektierten Zellen als radiale Gliazellen identifiziert wurden, lässt zudem den Rückschluss zu, dass mindestens eine Subpopulation der radialen Gliazellen Stammzelleigenschaften aufweist.

Bei ausdifferenzierten primären Neurosphären konnten Neurone, Astrozyten, Oligodendrozyten sowie radiale Gliazellen und undifferenzierte Vorläuferzellen festgestellt werden (siehe Figs. 15 und 17, Beispiel 8C). Insgesamt zeigten die Differenzierungsanalysen, dass die Neurosphären, die aus den 473HD-selektierten Zellen entstehen, sich zu unterschiedlichen Zelltypen ausdifferenzieren und somit Rückschlüsse auf das vorhandene Multipotential der einzelnen DSD-1-PG-exprimierenden Zellen erlauben.

Die Hybridomzelllinie 473HD wurde am 21.07.2004 unter der Bezeichnung DSM ACC2671 bei der DSMZ - Deutsche Sammlung von Mikroorganismen und Zellkulturen GmbH - Mascheroder Weg 18, D-38124 Braunschweig, hinterlegt.

Die Erfindung wird anhand der nachfolgenden Beispiele näher erläutert. Diese schränken den Schutzbereich der Erfindung jedoch nicht ein.

Beispiele

<u>Materialien und Geräte:</u> Deckgläser ⌀10mm (Marienfeld: 0111500), Fluoreszenzmikroskop Axioplan Imaging, Gewebekulturflaschen (Nunc: T25 136196), Gewebekulturschalen 100mm (Falcon: Typ 1029), Gewebekulturschalen 100mm (Falcon: Typ 3003), Gewebekulturschalen 35mm mit vier Vertiefungen (Greiner: 627170), Gewebekulturschalen mit 96 Vertiefungen (Nunclon: 167008), Immumount (Thermo Shandon: 9990402), Inkubator 6 % $CO_2$ Heraeus BBD 6220, Inverses Mikroskop (Leica 090135.001), Kryotom (Modell CM 3050S-1-1-1 Leica), Objektträger Super Frost Plus (Menzelgläser: 041300), Stereomikroskop Leica MZ 6, Tissue-tec (Jung: 020108926), Zentrifuge Heraeus (Fabrikat-Nr.: 40206491), Zentrifugierröhrchen 15ml (Greiner: 188271)

<u>Lösungen. Medien und immunologische Reagenzien</u>

BSA: BSA (Sigma: A-8806) 2 mg/ml in sterilem Minimal essential Medium (MEM) (Sigma: M-7278), steril filtriert.
Differenzierungsmedium: Dulbecco's Modified Eagle's Medium (Sigma: D-6546) und F12 (Sigma: N-4888) im 1:1 Verhältnis, plus L-Glutamin (Sigma: G-5763) 0,2 mg/ml, B27 (Gibco: 17504-044) 2 ml/100ml, Penicillin/Streptomycin (Gibco: 15140-122) 10 $\mu$l/ml und 1% (v/v) FCS. Lagerung bei 4°C maximal 2 Wochen.
FCS: 10% (v/v) FCS (Seromed: SO115/315X) in MEM (Sigma: M-7278) ansetzen und bei -20°C aufbewahren.
Krebs- Ringer- Hepes (KRH): 125 m M NaCl, 4,8 m M KCl, 1 ,3 m M $CaCl_2 \cdot 2H_2O$, 1 ,2 mM $MgSO_4 \cdot 7H_2O$, 1,2 mM $KH_2PO_4$, 5,6 mM D-Glucose, 25 mM N-2-hydroxy-ethylpiperazin-N' -2-ethansulfon Säure (HEPES); Lagerung bei -20°C. KRH/A: KRH mit 0,1% (v/v) BSA (Sigma: A-8806); Lagerung bei -20°C. Laminin: Laminin (steril, TEBU: 0172005) 25$\mu$g/ml in sterilem PBS; Lagerung bei -20°C.
Neurosphärenmedium (serumfreies Medium): Dulbecco's Modified Eagle's Medium (DMEM) (Sigma: D-6546) und F12 (Sigma: N-4888) im Verhältnis 1:1, plus L-Glutamin (Sigma: G-5763) 0,2 mg/ml, B27 (Gibco: 17504-044) 2 ml/100ml, Penicillin/Streptomycin (Gibco: 15140-122) 10 $\mu$l/ml. Lagerung maximal 2 Woche bei 4°C.
Ovomucoid: Trypsininhibitor (Sigma: T-6522) 1 mg/ml, BSA (Sigma: A-8806) 50 $\mu$g/ml und DNaseI (Worthington: LS02006) 40 $\mu$g/ml in L-15 Medium (Sigma: L-5520); Lagerung bei -20°C.
Paraformaldehyd (PFA): 4% (w/v) PFA in PBS (pH 7,4); Lagerung lichtgeschützt bei 4°C.
Phosphate Buffer Saline (PBS) pH 7,4: 137 mM NaCl, 3,0 mM KCl, 6,5 mM $Na_2HPO_4 \cdot 2H_2O$, 1,5 mM $KH_2PO_4$.
PBS/A: PBS mit 0,1% (w/v) BSA (Sigma: A-8806); Lagerung bei -20°C.
PBS 0,2% BSA: PBS mit 0,2% (w/v) BSA (Sigma: A8806); Lagerung bei 4°C maximal 4 Wochen.
PBS/Glycerin: PBS und Glycerin 1 :1 ; Lagerung bei 4°C.
PBS 1,7% NaCl: PBS (pH 7,4) mit 0,8% (w/v) NaCl; Lagerung lichtgeschützt bei Raumtemperatur (RT).
4% PFA: 90 ml Aqua bidest. wurden auf 66°C erhitzt und 4 g PFA zugegeben. Damit sich das PFA schneller löste, wurde durch 1-2 Tropfen 1 N NaCl ein leicht alkalischer pH-Wert erzeugt. Die Lösung wurde filtriert und mit 10 ml 10-fach PBS pH 7,4 auf das endgültige Volumen aufgefüllt. Der pH-Wert wurde auf pH 7,4 eingestellt.
Saccharose: 30% (w/v) Saccharose in Aqua dest.; Lagerung bei 4°C bis zu 2 Wochen.
Tris-HCl 50 mM (pH 9,5): 3,02 g Tris-Base in 500 ml Aqua dest.; pH-Wert mit 1 N HCl eingestellt; Lagerung bei 4°C.
Trypsin-EDTA (0,1 %): Trypsin- EDTA (Gibco: 25300-062)
Wachstumsfaktoren: EGF human recombinant (Preprotech: 100-15), bFGF (Preprotech: 100-18B) und Heparin (Sigma: H-3149) als essentieller aktivierender Co-Faktor des bFGF.
Primäre Antikörper: anti-βIII-Tubulin (Sigma: T-8660, Maus IgG, monoklonaler Antikörper), anti-GFAP (DAKO: Z-0334, Kaninchen, polyklonaler Antikörper), anti-GLAST (Kaninchen, polyklonaler Antikörper), anti-Nestin (Chemicon Internation: 353, Maus IgG, monoklonaler Antikörper), anti-Vimentin (Sigma: V 5255, Maus IgM, monoklonaler Antikörper), anti-RC2 (Developmental Studies Hybridoma Bank, Maus IgM, monoklonaler Antikörper), 04 (Maus IgM, monoklonaler Antikörper), 473HD (Ratte IgM, monoklonaler Antikörper).
Sekundäre Antikörper und Fluorochrome: anti-Kaninchen biotinyliert (a-rb biot.) (Amersham: RPN 1004), anti-Kaninchen CY2 (a-rb CY2) (Dianova: 111-225-003), anti-Maus biotinyliert (a-m biot.) (Amersham: RPN 1001), anti-Maus CY2 (a-m CY2) (Dianova: 115-225-044), anti-Maus CY3 (a-m CY3) (Dianova: 115-165-044), anti-Ratte biotinyliert (a-r biot.) (Jackson Immuno Reserch: 112-066-020), anti-Ratte CY3 (a-r CY3) (Dianova: 112-165-075), Streptavidin CY3 (Dianova: 016-160-084), Streptavidin FITC (Amersham: RPN 1232)

<u>Beispiel 1 : Herstellung des Antikörpers 473 HD</u>

A. Immunogen: Das Hybridom 473HD wurde im Rahmen eines Fusionsexperiments erzeugt. Weibliche Lou x SD Ratten wurden mit einer Glykoproteinfraktion des Maus-Nervensystems immunisiert. Diese Glykoproteinfraktion ist unter der Bezeichnung "Rest-L2" zusammengefasst.
"Rest-L2" umfasst Glykoproteine und Proteoglykane des adulten Zentralnervensystems der Maus: Adulte Gehirne wurden zunächst in hypotonem Puffer homogenisiert und die Membranfraktionen durch differentielle Zentrifugation gesammelt. Diese Präparationen adulter Maushirnmembranen wurden in der Folge mit Detergens solubilisiert und die unlöslichen Anteile wurden bei 100.000 g abzentrifugiert. Die im Überstand gelösten Proteine wurden anschließend über vier nacheinander geschaltete Affinitätssäulen aufgetrennt. Die Säule 1 war mit Ratten-Immunoglobulin gekoppelt (aus Präimmunserum gewonnen, unspezifische Negativkontrolle, 3 mg IgG pro ml feuchtem Affinitätsgel); die Säule 2 war mit dem monoklonalen Antikörper 324 (Rathjen & Schachner, EMBO J. 3:10 (1984)) gekoppelt, der mit dem Zelladhäsionsmolekül L1 reagiert (3 mg pro ml Feuchtgel); die Säule 3 war mit dem monoklonalen Antikörper H28.123 (Hirn et al., Brain Res. 214:433-439 (1981)) derivatisiert (3 mg pro ml Feuchtgel), der das Zelladhäsionsmolekül N-CAM erkennt; und die 4. Säule war mit dem monoklonalen Antikörper 336 (Noronha, A. B. et al., Brain Res. 385:237-244 (1986)) derivatisiert; dieser Antikörper reagiert mit einem spezifischen, N-gebundenen Kohlenhydrat des Nervensystems, das L2/HNK-1-Epitop genannt wird. Nach Beladung wurden die Säulen mit 30 Säulenvolumina Extraktionspuffer (50 mM Tris-HCl, 150 mM NaCl (TBS), 1 mM EDTA, 40 U Aprotinin/ml, 1 % NP-40, pH 7,4), anschliessend mit 10 Säulenvolumina des Extraktionspuffers mit 1,0 M NaCl anstelle von 150 mM NaCl, dann nochmals mit 10 Säurevolumina Extraktionspuffer gewaschen. Darauf erfolgte die Elution mit 15-20 ml 0,1 M Diethylamin, 1 mM EDTA, 0,5 % NP-40, pH 11,5. Das Eluat wurde mit 1,0 M Tris-HCl, pH 8,0, heruntergepuffert. Durch Druckdialyse unter 5 atm. $N_2$ in einer Amicon-Kammer über XM-50-Filter (Amicon Corp.) wurde das Eluat aufkonzentriert. Hierbei wurde die Detergens-Konzentration durch Zugabe lipophiler Matrix (SM2-Bio-Beads, Biorad) reduziert. Die Endkonzentration der gewonnenen Proteine lag bei 30-80 μg/ml.

Die resultierenden Glykoproteinfraktionen wurden weiter analysiert. Das Eluat der vierten Säule enthält bei dieser Konfiguration alle Glykoproteine und Proteoglykane des Zentralnervensystems der Maus, die das L2/HNK-1-Epitop enthalten, mit Ausnahme der durch die vorgeschalteten Säulen entfernten Adhäsionsmoleküle L1 und N-CAM. Diese beiden Moleküle treten in mehreren Formen im Zentralnervensystem der Maus auf, gehören in die Immunglobulin-Superfamilie der Zelladhäsionsmoleküle und tragen ebenfalls das L2/HNK-1-Epitop.

Die im Eluat der Säule 4 enthaltene Glykoprotein-Fraktion wurde "Rest L2" genannt und zur Immunisierung verwendet. Diese Fraktion enthielt die bekannten Antigene J1-160-Glykoprotein (entspricht Tenascin-R) und MAG ("myelinassociated glycoprotein") sowie Spuren DSD-1-PG/Phosphacan (Kruse, J. et al., Nature 311 :153-155 (1985)).

B. Immunisierung: Weibliche Lou x SD Ratten wurden mit je 50 μg "Rest-L2" multipel subkutan immunisiert, zunächst in komplettem, dann 2mal in inkomplettem Freund's Adjuvans in 14tägigen Abständen. Eine Woche nach der 3. Immunisierung wurden 50 μg "Rest-L2" in PBS intraperitoneal injiziert. Nach weiteren vier Tagen wurde die Ratte euthanasiert, die Milz entnommen und in Suspension überführt.

C. Fusion und Klonierung: Die Suspension wurde mit dem Mausmyelom X-Ag8-653 unter Verwendung von Polyethylenglykol (PEG 4000) fusioniert. Die Fusion wurde nach etablierten Standards durchgeführt und die resultierenden Hybridome wurden zunächst in RPMI/HAT, 20 % FCS nach der Methode der limiterenden Verdünnung in 2000 Einzelnäpfen (Mikrotiterplatten) kultiviert. Die Überstände wurden nach 10 Tagen per ELISA getestet und die mit dem Immunogen "Rest-L2" reagierenden Klonüberstände registriert. Die entsprechenden Klone wurden hochgezogen und die produzierten Überstände weiter geprüft. Der Vorläufer des Klons 473 wurde ausgesucht, da der Überstand bei immunzytologischen Untersuchungen an diversen Maushirnkulturen, die unterschiedliche Zeit in Kultur gehalten wurden, mit den Oberflächen unreifer Gliazellen und weiterer, nicht näher charakterisierter nicht-neuronaler Zellen reagierte. Der Klon wurde zweimal durch limitierende Dilution kloniert und erhielt die Nummer 473. Der Klon produzierte ein IgM der Ratte, welches mit der Oberfläche unreifer Gliazellen, insbesondere mit Untergruppen der unreifen Astrozyten und Oligodendrozyten reagierte. Diese Ergebnisse sowie die präliminäre Charakterisierung des Antigens wurden in einer Kurzmitteilung des Jahres 1988 anlässlich der Jahrestagung der American Society for Neuroscience präsentiert (Faissner, A., Soc. Neurosci. Abstr. 14: 920 (1988)).

D. Weiterentwicklung des Klons und Gewinnung von 473HD: Bei dem unter C beschriebenen Hybrid handelte es sich um eine Fusion zwischen Maus-Myelomzellen und Rattenmilzzellen, also einem Hybrid zwischen zwei Spezies. Diese Hybride sind essentiell instabil. Deshalb erwies es sich 1991 als notwendig, das Hybridom erneut zu klonieren. Hierzu wurden eingefrorene, bewahrte Klone der ursprünglichen Fusion wieder aufgetaut, nach den oben genannten Kriterien geprüft und der Klon 126-2E3 als Quelle eines in operationaler Hinsicht äquivalenten Antikörpers ausgemacht. In der Folge wurde dieser Klon durch das Verfahren der limitierenden Dilution zweimal kloniert und 473HD genannt. Der entsprechende Antikörper besaß die gleichen Eigenschaften wie der durch den Klon 473 (Faissner, A., Soc. Neurosci Abstr. 14:920 (1988)) produzierte Antikörper. Die Hybridomzelllinie 473HD wurde unter der Bezeichnung DSM ACC2671 hinterlegt.

Beispiel 2: Erstellung von Gefrierschnitten

Für die hier beschriebenen Experimente wurden zeitlich angepaarte Mäuse des Stammes NMRI, die bei der Firma Charles River erworben wurden, verwendet. Für immunhistochemische Analysen *in vivo* wurden bei Embryonalstadien E10-18 mindestens drei entnommene Embryonen pro Stadium dekaptiert und fixiert, um Schnitte in sagittaler, transversaler und frontaler Orientierung anzufertigen.

A. Präparation und Kryoprotektion: Eine tragende Maus des Stammes NMRI wurde durch $CO_2$ betäubt und durch zervikale Dislokation getötet. Nach dem Öffnen der Bauchhöhle wurden Embryonen dem Uterus entnommen und in eine Petrischale mit PBS überführt. Unter dem Stereoskop wurden die embryonalen Entwicklungsstadien nach den Kriterien von Theiler bestimmt und die Embryonen wurden dekaptiert. Die Köpfe wurden sofort in 4% PFA überführt und 24 h bei 4°C inkubiert. Dieses Verfahren zur Gewebefixierung ermöglichte eine relativ gute Durchdringung und Erhaltung des Gewebes, wobei durch das PFA die Proteine immobilisiert wurden.

Das über Nacht immersionsfixierte Gewebe wurde schliesslich 24 h bei 4°C in 30% Saccharose dehydriert. Danach wurde das Gewebe auf Alufolie überführt, die restliche Flüssigkeit mit einem Papiertuch entfernt und das Gewebe auf Trockeneis in ein für Kryoprotektion geeignetes Medium (Tissue-tec) eingebettet. Das nach diesem Verfahren fixierte und eingebettete Gewebe wurde bei -80°C gelagert.

B. Herstellung von Gefrierschnitten: Für embryonales Hirngewebe wurden -20°C Kammertemperatur und -18°C Objekttemperatur eingestellt. Das eingebettete Gewebe wurde mit Tissue-tec auf dem Objekttisch befestigt und ca.1h auf die Kammer/Objekttemperatur äquilibriert.

Es wurden 16 μm dicke Schnitte angefertigt. Die Schnittebene bzw. -orientierung wurde mikroskopisch kontrolliert, falls notwendig wurde die Einstellung des Objekttisches nachkorrigiert. Die Schnitte wurden auf an die Kammertemperatur äquilibrierte Objektträger aufgezogen und ca. 1h bei Raumtemperatur (RT) getrocknet. Danach wurden die Präparate in Objektcontainern bei -20°C gelagert. Das Aufbewahren über einen längeren Zeitraum erfolgte bei -80 °C.

Beispiel 3. Immunhistochemische Färbung von Gewebepräparaten

Die Co-Lokalisation des 473HD-Epitopes wurde durch immunhistochemische Verfahren untersucht. Dazu wurde eine Doppelimmunfluoreszenzanalyse mit den entsprechenden primären und sekundären Antikörpern durchgeführt. Als zelltypspezifische Marker dienten GFAP für Astrozyten (Bignami, A. et al., Brain Res. 43:429-435 (1972); Levitt, P., Rakic, P., Comp. Neurol. 193:815-840 (1980); Dahl D. et al., J. Comp. Neurol. 239:75-88 (1985)), βIII-Tubulin für Neurone (Lee, M. K. et al., Cell Motil. Cytoskeleton 17:118-132 (1990)), 04 für Oligodendrozyten (Sommer I., Schachner, M., Dev. Biol. 83:311-327 (1984)), RC2 für radiale Gliazellen (Mission, J. P. et al., Dev. Brain Res. 44:95-108 (1988); Edwards, M. A. et al., Neurosci. 36:12-144 (1990); Malatesta, P. et al., Development 127:5253-5263 (2000); Malatesta, P. et al., Neuron. 37:751-764 (2003); Hartfuss, E. et al., Developmental Biol. 229:15-30 (2001)) und Nestin für Vorläuferzellen (Frederiksen, K., McKay, R. D. G., J. Neurosci. 8:1144-1151 (1988); Lendahl, U., McKay, R. D. G., Trends Neurosci. 13:132-137 (1990)). Für die Detektion der radialen Gliazellen wurden RC2 (Mission, J. P. et al., Dev. Brain Res. 44:95-108 (1988); Edwards, M. A. et al., Neurosci. 36:12-144 (1990); Malatesta, P. et al., Development 127: 5253-5263 (2000); Malatesta, P. et al., Neuron 37:751-764 (2003); Hartfuss, E. et al., Developmental Biol. 229:15-30 (2001)), Glast (Shibata, T. et al., J. Neurosci.17:9212-9219 (1997)) und Vimentin (Pixley, S. K., de Vellis, J., Brain Res. 317:201-209 (1984); Meyer, S. A. et al., J. Neurosci. Res. 24:251-259 (1989); Monzon-Mayor, M. et al., J. Comp. Neurol. 295:569-579 (1990); Oudega, M., Marani, E., J. Anat. 179:97-114 (1991)) als Marker eingesetzt (Antikörper-Übersicht siehe Tab. 1).

Die bevorzugt verwendeten Fluorochrome FITC, CY2 und CY3, die als Marker an über Streptavidin an die sekundäre Antikörper gekoppelt wurden, zeigen bei Anregung im UV-Licht mit entsprechenden Anregungsfiltern eine gelbgrüne (FITC, CY2) oder rote (CY3) Fluoreszenz und machen die Antigen-Antikörper-Komplexe sichtbar.

Tab. 1: Primäre und sekundäre Antikörper, die für die Charakterisierung der Stammzellen im Differenzierungsassay verwendet wurden

| Primäre Antikörper | Spezies | Subtyp | Epitop | Lokalisation des Epitopes; Detektierter Zelltyp bzw. molekulare Struktur | Sekundäre Antikörper zur Detektion |
|---|---|---|---|---|---|
| anti-GFAP | Kaninchen | | Protein | Intermediärfilament von Astrozyten | anti-rb Ig CY2 oder anti-rb Ig biot.+ Streptavidin FITC |

(fortgesetzt)

| | | | | | |
|---|---|---|---|---|---|
| anti-βIII-Tubulin | Maus | IgG | Protein | Mikrotubuli postmitotischer Neurone | anti-m Ig CY3 oder anti-m Ig biot.+ Streptavidin FITC |
| anti-O4 | Maus | IgM | Kohlenhydrat | Glykolipid von Oligodendrozyten | anti-m Ig CY3 oder anti-m Ig biot+ Streptavidin FITC |
| anti-Nestin | Maus | IgG | Protein | Intermediärfilament undifferenzierter Vorläufer | anti-m Ig CY3 oder anti-m Ig biot+ Streptavidin FITC |
| anti-Glast | Kaninchen | | Protein | Glutamattransporter auf radialen Gliazellen | anti-rb Ig CY2 oder anti-rb Ig biot.+ Streptavidin FITC |
| anti-Vimentin | Maus | IgM | Protein | Intermediärfilament von radialen Gliazellen | anti-m Ig CY3 oder anti-m Ig biot+ Streptavidin FITC |
| anti-RC2 | Maus | IgM | Protein | Intermediärfilament von radialen Gliazellen | anti-m Ig CY3 oder anti-m Ig biot+ Streptavidin FITC |
| 473HD | Ratte | IgM | Kohlenhydrat | Phosphacan, RPTPβ; ECM | anti-r Ig CY3 oder anti-r Ig biot+ Streptavidin FITC |

Zu Beginn wurde der Boden einer Färbekammer mit Aqua dest.-feuchten Filterpapierstreifen ausgelegt. Die ca. 5 min auf Raumtemperatur äquilibrierten Schnittpräparate wurden in der Färbekammer platziert. Sie wurden zunächst in PBS 1,7% NaCl mit 10% Serum (4% Ziegeserum + 3% Schafserum + 3% Eselserum) 1h bei RT rehydriert. Pro Objektträger wurden 300 $\mu$l Lösung aufgetragen, um eine optimale Schnittflächenbedeckung zu gewährleisten. Die primären Antikörper wurden unmittelbar vor der Anwendung in PBS 1,7% NaCl 10% Serum verdünnt. Für die Doppelimmunfluoreszenzfärbung wurden in folgender Kombination 473HD (1:300) mit βIII-Tubulin (1:250), 473HD (1:300) mit GFAP (1:300), 473HD (1:300) mit 04 (1:100), 473HD (1:300) mit Nestin (1:1000), 473HD (1 :300) mit RC2 (1:500) eingesetzt. Es wurde bei 4°C über Nacht inkubiert. Kontrollen zur Identifizierung eventueller unspezifischer Bindungen der sekundären Antikörper wurden analog, jedoch ohne primären Antikörper angefertigt. Nach der Inkubation wurden die Objektträger in eine Glasküvette mit PBS überführt und dreimal je 5 min in PBS gewaschen. Die darauf folgende Inkubation mit sekundären Antikörpern dauerte 2h bei RT, wobei die sekundären Antikörper in PBS/A verdünnt eingesetzt wurden. Auf mit 473HD/βIII-Tubulin, 473HD/Nestin, 473HD/O4, 473HD/RC2 vorinkubierten Schnitten wurden anti-r Ig CY3 (1:500) und anti-m Ig biot. (1:500), auf mit 473HD/GFAP vorinkubierten Schnitten anti-r Ig CY3 (1:500) mit anti-rb Ig biot (1 :500) aufgetragen. Die Kontrollen wurden mit jedem verwendeten sekundären Antikörper separat behandelt. Nach der Inkubation mit dem sekundären Antikörper wurden die Schnittpräparate wiederholt in PBS gewaschen. Da an die biotinylierten sekundären Antikörper ein Fluorochrom gekoppelt wurden sollte, inkubiert man alle Objekte, außer dem CY3-Kontrollobjekt, mit Streptavidin FITC (1:500) 30 min bei Raumtemperatur. Gleichzeitig wurde ein Hoechst-Farbstoff ($1:10^5$) zugegeben, der in die DNA interkaliert und unter UV-Anregung die Zellkerne sichtbar macht. Es wurde erneut wie oben gewaschen, bevor die Objekte im Immumount mit Deckgläsern eingedeckelt wurden. Die Objekte wurden mit UV-Epifluoreszenz (Axioplan II) mit geeigneten Anregungsfiltern mikroskopiert, dokumentiert und ausgewertet.

Beispiel 4: Primäre Einzelzellsuspensions-Herstellung, Immunopanning und Neurosphärenkultivierung

A. Herstellung einer primären Einzelzellsuspension: Die Zellkulturexperimente wurden mit Gewebe aus kompletten Vorderhirnen von vier entnommenen Embryonen durchgeführt. Die Dekaptierung der Embryonen erfolgte wie in Beispiel 2A beschrieben. Die Vorderhirne wurde in einer Petrischale mit MEM herauspräpariert und anschließend in ein Eppendorfgefäß mit 1ml MEM überführt. Es folgte die enzymatische Gewebedissoziation, wobei zuerst das MEM durch 500 $\mu$l 0,1% Trypsin-EDTA kurz ersetzt wurde und die Gewebe weitere 8 min bei 37°C in 1 ml 0,1% Trypsin-EDTA inkubiert wurden. Danach wurde das Gewebe in ein Zentrifugierröhrchen überführt und der proteolytische Gewebeverdau durch Zugabe von 1ml Ovomucoid gestoppt. Anschließendes 10-maliges Triturieren mit einer 1000 $\mu$l-Mikropipettenspitze ergab eine Einzelzellsuspension, die durch Zentrifugation für 5 min bei 1000 rpm (215 g) und 22°C pelletiert wurde. Nach der Zentrifugation wurde der Überstand bis auf 0,5 ml entfernt und durch 0,5 ml MEM ersetzt. Nach dem Resuspendieren der Zellen wurde die Zellzahl pro ml mittels einer Neubauer-Zählkammer bestimmt und die Zellen wurden in der gewünschten Konzentration ausplattiert.

B. Immuncytochemische Analyse akut dissoziierter Zellen: Diese Analyse diente der Identifizierung der Zelltypen in einer Einzelzellsuspension. Die Methode basiert auf dem Prinzip von Beispiel 3.

Zwei sterile Gewebekulturschalen ($\varnothing$35 mm) mit vier Vertiefungen wurden mit 100 $\mu$l/Vertiefung Poly-DL-Ornithin (0,5 $\mu$g/ml in Aqua bidest.) 1h bei 37°C beschichtet, anschließend zweimal mit Aqua bidest. ausgewaschen und bei RT getrocknet. Auf die getrockneten Vertiefungen wurde 100 $\mu$l/Vertiefung Laminin aufgetragen und eine weitere Stunde bei 37°C inkubiert. Nach der Inkubation wurden die Vertiefungen zweimal mit PBS ausgewaschen und sofort mit Differenzierungsmedium (50 $\mu$l/Vertiefung) aufgefüllt. $2,5 \times 10^3$ Zellen pro Vertiefung wurden aus der resuspendierten Zellsuspension ausplattiert und 1 h bei 37°C inkubiert. Anschließend wurde das Differenzierungsmedium entfernt und die Zellen wurden 5 min bei RT in 75 $\mu$l KRH/A pro Vertiefung inkubiert. Danach wurde für 30 min bei RT mit Antikörper 473HD (1 :250 in KRH/A) und Antikörper 04 (1:25 in KRH/A) inkubiert. Die Zellen wurden 5 min in KRH gewaschen und 15 min bei RT mit 4% PFA fixiert. Nach zweimaligem Waschen mit PBT1 bzw. bei 473HD/O4 mit PBS/A wurden die Zellen für 30 min mit $\beta$III-Tubulin (1 :250), GFAP (1 :250), Glast (1:1000), Nestin (1:1000), RC2 (1:250) und Vimentin (1:200) in PBT1 inkubiert. Anschließend wurde zweimal in PBS/A gewaschen und wiederum 30 min bei RT mit dem sekundären Antikörper inkubiert. Dabei wurden auf 473HD/$\beta$III-Tubulin, 473HD/Nestin, 473HD/RC2, 473HD/Vimentin und 473HD/O4 der a-r Ig CY3 (1 :500) und a-m Ig biot. (1 :500) und auf 473HD/GFAP und 473HD /Glast die a-r Ig CY3 (1:500) und a-rb Ig biot. (1:500) aufgetragen und nach 30 min Inkubation erneut in PBS/A zweimal ausgewaschen. Darauf folgend wurden die Zellen 20 min bei RT mit 1:500 verdünntem Streptavidin FITC und $1:10^5$ verdünntem Hoechst in PBS/A inkubiert. Nach 3-maligem Waschen in PBS wurden die Zellen in ca. 20$\mu$l PBS/Glycerin aufgenommen und mit UV-Epifluoreszenz und geeigneten Anregungsfiltern mikroskopiert.

C. Immunopanning: Eine sterile Gewebekultur-Petrischale (100 mm, Falcon: 1029) wurde über Nacht bei 4°C mit polyklonalem anti-r Ig biot. (50 $\mu$g in 10 ml 50 mM Tris-HCl, pH 9,5) beschichtet und anschließend dreimal mit 8 ml PBS gewaschen. Danach wurde die Petrischale 1 h bei RT mit monoklonalen 473HD Antikörpern (1:500 in 5 ml PBS 0,2% BSA) inkubiert. Während dieser Inkubation wurden die Zellen ($1,6 \times 10^6$) aus einer primären Einzelzellsuspension (Beispiel 3A) in 10 ml MEM 0.2% BSA aufgenommen und 1h bei RT präinkubiert. Diese Präinkubation war notwendig, da frühestens 1h nach der Dissoziation durch Trypsin wieder 473HD-Epitopexpression an den isolierten Vorderhirnzellen nachgewiesen werden konnte.

Nach der Inkubation mit Antikörper 473HD wurde die Petrischale dreimal mit 8 ml PBS gewaschen. Unmittelbar danach wurden $1,6 \times 10^6$ Zellen in 10 ml MEM 0,2% BSA auf der beschichteten Schale ausplattiert und 1 h bei RT inkubiert.

Danach wurde die ungebundene, immunnegative Zellfraktion entfernt, und restliche, nicht adhärierte Zellen wurden durch achtmaliges Waschen mit je 8 ml PBS entfernt. Die adhärierten Zellen wurden mit 5 ml Trypsin-EDTA 6 min bei 37°C, 6% $CO_2$ inkubiert und dadurch abgelöst. Die Trypsinisierung wurde anschließend mit 5 ml MEM 10% FCS gestoppt. Die Zellsuspension wurde in ein Zentrifugierröhrchen übertragen und die Zellen durch Zentrifugation (10 min bei 800 rpm (140 g), RT) pelletiert. Nach der Zentrifugation wurde der Überstand bis auf 0,5 ml entfernt und durch Neurosphärenmedium ersetzt. Nach dem Resuspendieren der Zellen wurde die Zelldichte mittels einer Neubauer-Zählkammer bestimmt und die Zellen wurden in der gewünschten Konzentration ausplattiert.

Die Anreicherung der über den Antikörper 473HD selektiv isolierten Zellpopulation wurde immuncytochemisch analysiert.

D. Neurosphärenkulturen in serumfreiem Medium:

Primäre Neurosphären: Die nach Trypsinierung gewonnene Einzelzellsuspension aus Bsp. 4A bzw. die durch Immunopanning isolierte Zellpopulation aus Bsp. 4C wurde nach Zentrifugieren für 10 min bei 1000 rpm (215 g) in Medium resuspendiert. Nach Bestimmung der Zelldichte wurde die Einzelzellsuspension mit definierter Zelldichte pro ml Neurosphärenmedium ausplattiert:

- 100.000 Zellen/ml in 4 ml Neurosphärenmedium mit bFGF (20 ng/ml), EGF (20 ng/ml) und Heparin (0,5 U/ml) in einer Zellkulturflasche T25;
- 5.000 Zellen/ml in 2 ml Neurosphärenmedium mit bFGF (20 ng/ml), EGF (20 ng/ml), Heparin (0,5 U/ml) in einer Gewebekulturschale Ø 35 mm;
- 100 Zellen/ml in 100 $\mu$l Neurosphärenmedium mit bFGF (20 ng/ml), EGF (20 ng/ml) und Heparin (0,5 U/ml) in Vertiefungen einer Mikrotiterplatte mit 96 Vertiefungen;
- 1-10 Zellen in 100 $\mu$l Neurosphärenmedium mit bFGF (20 ng/ml), EGF (20 ng/ml) und Heparin (0,5 U/ml) in Vertiefungen einer Mikrotiterplatte mit 96 Vertiefungen.

Das Neurosphärenmedium wurde vor dem Experiment angesetzt. Die Wachstumsfaktoren (aus 1000x Stammlösung) und Heparin (aus 1000x Stammlösung) wurden unmittelbar vor dem Ausplattieren dem Medium zugesetzt. Die Zellkulturen wurden bei 37°C, 6 % $CO_2$ inkubiert.

Unter serumfreien Bedingungen können neurale Zellen bis zu 14 Tagen oder länger kultiviert werden. Der in dem Medium vorhandene pH-Indikator Phenolrot ermöglicht die optische Kontrolle des pH-Wertes, da nur ein im physiologischen Bereich liegender pH-Wert des Mediums die optimalen Wachstumsbedingungen ermöglicht.

Auch ohne Farbumschlag wurden die Zellkulturen nach 3-4 Tage routinemäßig passagiert. Für das Passagieren wurde das Gesamtvolumen der Zellkultur in Zentrifugierröhrchen überführt, 5 min bei 800 rpm (140 g) zentrifugiert und pelletiert. Der Überstand wurde bis auf 1ml entfernt, das Pellet durch vorsichtiges Triturieren resuspendiert und in Neurosphärenmedium mit Wachstumsfaktoren, wie vorstehend beschrieben, erneut ausplattiert.

<u>Sekundäre Neurosphären:</u> Um den Stammzellcharakter der Zellen in den Neurosphärenkulturen zu überprüfen, wurden sekundäre Neurosphärenkulturen angelegt. Dazu wurden primäre Neurosphärenkulturen in Zentrifugierröhrchen übertragen und 5 min bei 800 rpm (140 g) zentrifugiert. Der Überstand wurde entfernt und das Pellet in 1 ml Medium trituriert. Die dadurch gewonnene Einzelzellsuspension wurde in gleicher Zelldichte wie die Zellen bei Bildung primäre Neurosphären (1x10$^5$ Zellen/ml) in das serumfreie Medium mit Wachstumsfaktoren übertragen und bei 37°C, 6 % $CO_2$ inkubiert. Nach 24 h konnte der Beginn der Bildung sekundärer Neurosphären beobachtet werden. Der Ansatz zeigt, dass in den primärem Neurosphären Stammzellen enthalten waren, die sich unter den angegebenen Bedingungen subkultivieren lassen. Das Kulturverfahren führt also nicht zum Verlust der Stammzelleneigenschaften.

<u>E. Differenzierungsanalyse der Neurosphärenkulturen:</u> Die Differenzierungsanalyse wurde im 24 h Intervall bis zum 7.Tag *in vitro* (7div) durchgeführt.

Zwei sterile Gewebekulturschalen (Ø 35 mm) mit vier Vertiefungen wurden mit 100 $\mu$l Polyornithin (10 $\mu$g/ml) pro Vertiefung 1 h bei 37°C beschichtet und anschließend zweimal mit Aqua ster. gewaschen. Auf den luftgetrockneten Vertiefungen wurde pro Vertiefung 100 $\mu$l Laminin (10 $\mu$g/ml) aufgetragen und 1 h bei 37°C inkubiert. Nach der Inkubation wurde zweimal mit PBS gewaschen und sofort mit 50 $\mu$l Differenzierungsmedium pro Vertiefung aufgefüllt, um das Austrocknen zu vermeiden.

Pro Vertiefung wurde je eine primäre oder sekundäre Neurosphäre unter dem Stereoskop mit der Mikropipette übertragen und 1 h bei 37°C, 6% $CO_2$ zur Adhäsion an das Substrat stehen gelassen. Anschließend wurden die Kulturen mit 1,5 ml Differenzierungsmedium geflutet und bei 37°C, 6% $CO_2$ bis zur immuncytochemischen Analyse inkubiert.

Zur immuncytochemischen Färbung wurde das Differenzierungsmedium entfernt und 5 min bei RT mit 75 $\mu$l KRH/A pro Vertiefung gewaschen. Danach wurde - falls gewünscht - für 30 min bei RT mit Antikörper 473HD (1 :300 in KRH/A) und 04 (1:25 in KRH/A) inkubiert. Die Neurosphären wurden 5 min in KRH gewaschen und 20 min bei RT mit 4% PFA fixiert. Nach der Fixierung folgte 2-maliges Waschen mit PBT1 bzw. bei mit 473HD und 04 präinkubierten Neurosphären mit PBS/A. Die Neurosphären wurden für 30 min mit βIII-Tubulin (1:250), GFAP (1:300), Glast (1:1000), Vimentin (1:200) und Nestin (1:1000) in PBT1 inkubiert. Anschließend wurden die Objekte zweimal in PBS/A gewaschen und wiederum 30 min bei RT mit dem sekundären Antikörper inkubiert. Dabei wurden auf die mit βIII-Tubulin, Nestin und 04 präinkubierten Neurosphären a-m Ig biot. (1:500), auf die mit 473HD präinkubierten Neurosphären a-r IgM CY3 (1:500) und auf die mit GFAP und Glast präinkubierten Neurosphären a-rb Ig biot. (1 :500) aufgetragen. Nach der Inkubation wurde erneut in PBS/A zweimal gewaschen. Darauf folgend wurden die Neurosphären 30 min bei RT mit 1: 500 verdünntem Streptavidin CY3 und 1:10$^5$ verdünntem Hoechst in PBS/A inkubiert. Nach dreimaligem Waschen in PBS wurden die Neurosphären in ca. 20 $\mu$l PBS/Glycerin aufgenommen und mit UV-Epifluoreszenz bei entsprechenden Anregungsfiltern mikroskopiert.

<u>Beispiel 5: Auswertungsmethoden</u>

Alle immunhistologischen und immuncytochemischen Daten wurden mit Hilfe des Epi-Fluoreszenzmikroskops und des dazu gehörenden Bildschirmanalyseprogramms Axiovision ausgewertet. Bei der Verwendung von UV-Anregungsfiltern wurden die Immunfärbungen bei 40x Vergrößerung unter gleicher Belichtungszeit (für CY3 1000 ms, für FITC bzw. CY2 700 ms und für DAPI 7 ms) dokumentiert. Die Ergebnisse der immuncytochemischen Methoden wurden an mindestens 150 Zellen pro Färbung in Anzahl der positiven Zellen pro Anzahl der gesamt gezählten Zellen zusammengefasst.

Die Neurosphärenkulturen wurden mit inversem Mikroskop (Olympus) bei 10 x und 40 x Vergrößerung und Carl Zeiss-Vision CD 58 dokumentiert.

Standardabweichungen unabhängiger Experimente ergaben sich nach folgender Formel:

$$s = \sqrt{1/n\text{-}k\left[s_1^2(n_1\text{-}1) + s_2^2(n_2\text{-}1) + .. s_k^2(n_k\text{-}1)\right]}$$

Die Standardabweichung des Mittelwertes wurde nach der Formel $SEM^2 = s^2/n$ berechnet (s = Standardabweichung, n = Anzahl der Messwerte, k = Anzahl der Stichproben, SEM = Standardfehler des Mittelwertes).

Beispiel 6: Ergebnisse der Doppelimmunfluoreszenzanalyse an frontalen Schnitten des cerebralen Kortex *in vivo*

Die Mehrkanalaufnahme in Fig. 4 zeigt, dass das immunreaktive spezifische Signal des 473HD-Epitopes zum Teil mit Nestin-positiven Zellen co-lokalisiert ist.

Durch die Kombination der RC2- und 473HD-Antikörper bei der Doppelimmunoanalyse wurde es möglich, nicht nur die radialen Gliazellen zu markieren, sondern auch die Co-Lokalisation des 473HD-Epitops an den RC2-immunoreaktiven Zellen zu analysieren. Die Ergebnisse der Analyse zeigten eine stark verbreitete Expression des RC2-Epitopes im gesamten telencephalen Gewebe (Fig. 5). Dabei konnten in einzelnen Fällen sowohl die in der ventrikulären Zone liegenden Somata, als auch die radialen Zellfortsätze der radialen Gliazellen deutlich unterschieden werden. Es konnte im gesamten cerebralen Kortex und in der ventrikulären Zone der GE eine radial orientierte Lokalisation der RC2-Expression beobachtet werden. Hingegen sind in der subventrikulären Zone der GE und in der kortikalen Platte des cerebralen Kortex die RC2-markierten Zellen tangential orientiert. Interessanterweise zeigte die Lokalisation der RC2-positiven Zellen ein Orientierungsmuster, das dem Expressionsmuster des 473HD-Epitops identisch ist.

Die Doppelimmunfärbung zeigte, dass auch im sich entwickelnden Telencephalon ein großer Teil der RC2-immunreaktiven Zellen das 473HD-Epitop exprimiert. In Fig. 5 ist exemplarisch eine Mehrkanalaufnahme der Doppelimmunoanalyse mit 473HD- und RC2-Antikörpern dargestellt.

Die postmitotischen Neurone wurden mit anti-βIII-Tubulin detektiert und sind bei E13 fast ausschließlich in der kortikalen Platte lokalisiert (Fig. 6). Obwohl auch das 473HD Epitop in der kortikalen Platte spezifische Signale aufweist, sind diese nicht an βIII-positiven Zellen co-lokalisiert. Die spezifischen Signale des 473HD-Epitopes erscheinen jedoch zwischen den βIII-positiven Zellen liegend und lassen sich im Einzelfall in ihrem radialen Verlauf bis zur basalen Oberfläche der ventrikulären Zone zurückverfolgen.

Die Analyse mit anti-GFAP und anti-O4 ergab bei E13 keine zelltypspezifischen immunpositiven Signale und demzufolge keine Co-Lokalisation mit dem 473HD-Epitop. Dies war zu erwarten, da die kortikalen Astrozyten erst ab E16 und die Oligodendrozyten um die Geburt bzw. in den ersten postnatalen Stadien entstehen (Temple, S., Nature 414:112-117, (2000); Qian, X. et al., Neuron 28:69-80 (2000)).

In Fig. 7 sind die Ergebnisse der Doppelimmunfluoreszenzanalyse mit dem Antikörper 473HD und anti-GFAP an frontalen Schnitten des cerebralen Kortex bei E18 dargestellt. Die fluoreszenzmikroskopischen Aufnahmen zeigen, dass die Zellkörper der GFAP-positiven Zellen sich noch überwiegend in der intermediären Zone und subkortikalen Platte befinden, wobei die Zellfortsätze ein ausgeprägtes Netzwerk fast im gesamten cerebralen Kortex ausbilden. Diese Analyse wurde als positive Kontrolle zur GFAP-Markierung bei E13 betrachtet und zeigt gleichzeitig, dass die Expression des 473HD-Epitopes in späteren Entwicklungsstadien an der Zellmembran der GFAP-positiven Zellen co-lokalisiert ist und somit mit den Astrozyten assoziiert.

Zusammenfassend lässt sich feststellen, dass das 473HD-Epitop nicht von differenzierten Zelltypen wie Neuronen, Astrozyten und Oligodendrozyten bei E13 exprimiert wird und dass ein Teil der Nestin-positiven Vorläufer und radialen Gliazellen 473HD positiv ist.

Beispiel 7: Selektive Isolierung einer 473HD-positiven Zellpopulation aus einer Gewebeprobe

A. Detektion von 473HD-positiven Zellen in Kultur: Um den Anteil 473HD-positiver Zellen zu bestimmen, wurden akut dissoziierte Zellkulturen aus E13 Vorderhirn nach 2h in Kultur über indirekte Immunfluoreszenzfärbung mit Antikörper 473HD markiert. Da nach Verdau mit Papain nur wenige Zellen 473HD-immunpositiv waren, wurden alternative Dissoziierungsverfahren getestet. Eine Gewebedissoziation mit 0,1% Trypsin-EDTA zeigte überraschenderweise nach 1-stündiger Präinkubation vor dem Ausplattieren und anschließender Immunfärbung mit 473HD 4% 473HD-positive Zellen.

Die Ergebnisse einer immuncytochemischen Analyse mit Antikörper 473HD an isolierten Zellsuspensionen hängen nicht nur mit der Wirkungsweise des für die Gewebedissoziation eingesetzten Enzyms, sondern auch mit der Dauer der Inkubation vor Beginn der immunocytochemischen Analyse zusammen, da das 473HD-Epitop offensichtlich zunächst indirekt abgebaut wird.

Die Verlängerung der Inkubationszeit in MEM, 0,2% BSA auf 2h (1h bei Raumtemperatur und 1h 37°C) und die Reduktion der Trypsinierungsdauer auf 6 min bei 37°C erwiesen sich als geeignet für den quantitativen Nachweis der membranständigen Isoform DSD-1-PG/RPTPβ in Einzelzellsuspension. Unter diesen Bedingungen konnte der Anteil 473HD- positiver Zellen bei E13 Vorderhirn ermittelt werden: $21 \pm 4,9\%$ (n = 11) (Fig. 8).

Um die Validität dieser Bestimmung weiter zu verifizieren, wurde durch Verwendung zelltypspezifischer Marker das Im munoprofil der akut dissoziierten Zellpopulation ermittelt. Dabei wurde der Anteil undifferenzierter Vorläuferzellen, radialer Gliazellen und postmitotischer Neurone immuncytochemisch bestimmt.

Die Analyse ergab, dass in einer akut dissoziierten Einzelzellkultur aus Maus-Vorderhirn bei E13 $46 \pm 9,8$ % (n=11) Nestin-positive Vorläuferzellen vorhanden sind. Bei E13 Vorderhirn konnten $67 \pm 9,3$ % (n=6) RC2-positive, $40 \pm 12$ % (n= 11) Glast-positive und $42 \pm 7,9$ % (n= 10) Vimentin-positive Zellen detektiert werden. Neben diesen wurden $30 \pm 8,4$ % (n= 11) postmitotische Neurone und $3 \pm 1,5$ % (n= 6) GFAP-positive Zellen detektiert (Tab. 2).

Tab. 2: Ergebnisse der immuncytochemischen Analysen mit zelltypspezifischen primären Antikörpern an akut dissoziierten Zellpopulation aus Vorderhirm bei E13 nach 2h *in vitro* (n = Anzahl unabhängiger Experimente).

| Zelltypspezifische Marker | Anteil (%) positiver Zellen in der akut dissoziierten Zellpopulation | |
|---|---|---|
| Nestin | $46 \pm 9,8$ | (n=11) |
| RC2 | $67 \pm 9,3$ | (n=6) |
| Vimentin | $42 \pm 7,9$ | (n=10) |
| Glast | $40 \pm 12$ | (n=11) |
| βIII-Tubulin | $30 \pm 8,4$ | (n=11) |
| GFAP | $3 \pm 1,5$ | (n=6) |
| 473HD | $21 \pm 4,9$ | (n=11) |

B. Immunopanning: Die im embryonalen Vorderhirn einer Maus bei E13 das 473HD-Epitop exprimierenden Zellen sollten näher charakterisiert werden.

Das in Beispiel 3 beschriebene Immunopanning-Protokoll kann in dieser Form für die Isolierung der 473HD-Epitop exprimierenden Zellen aus embryonalem neuralem Gewebe verwendet werden.

Um die Effizienz und den Erfolg der Isolierung 473HD-positiver Zellen zu bewerten, wurde der Anteil immunpositiver Zellen vor und nach dem Immunopanning bestimmt (Fig. 10).

Die in Fig. 10 dargestellten fluoreszenzmikroskopischen Aufnahmen wurden quantitativ für ein Experiment ausgewertet. Vor dem Immunopanning exprimierten 54 von 246 Zellen und nach dem Immunopanning 203 von 225 Zellen das 473HD-Epitop. Es wurde also eine Anreicherung von 22% auf 90% erzielt. Die Ausbeute betrug $0,2 \times 10^6$ Zellen oder 57%.

Durch Immunopanning konnten aus einer Einzelzellsuspension primär isolierter Zellen aus E13 Vorderhirn $0,157 \pm 0,023 \times 10^6$ (n= 11) 473HD-Epitop exprimierende Zellen aus den $1,6 \times 10^6$ für das Immunopanning ausplattierten Zellen isoliert werden. Die Anreicherung betrug $90,16 \pm 6,9\%$ (n= 11), wobei der maximale erzielte Anreicherungswert bei 98,7% lag.

Beispiel 8: Charakterisierung DSD-1-PG/RPTPβ-exprimierender Zellen aus E13 Vorderhirn

A. Immuncytochemische Analyse der selektiv isolierten 473HD-positiven Zellpopulation: Eine Aussage über die Zusammensetzung der gemäß Bsp. 7B selektiv isolierten 473HD-positiven Zellpopulation wurde durch immuncytochemische Analyse 2h nach dem Immunopanning ermöglicht. Es wurden folgende zelltypspezifische Marker verwendet: Nestin, Glast, RC2, Vimentin und βIII-Tubulin. Dabei stand die Bestimmung des Anteils an undifferenzierten Vorläuferzellen, radialen Gliazellen und glialen Vorläufern sowie an postmitotischen Neuronen in der 473HD-positiven Zellpopulation im Vordergrund (Fig. 11). Die Ergebnisse zeigen, dass die angereicherte 473HD-positive Zellpopulation einen heterogenen Charakter aufweist. Es wurden in dieser Zellpopulation neben Nestin-positiven, undifferenzierten Vorläuferzellen (62%) ein großer Anteil RC2-positiver radialer Gliazellen (85%) detektiert. Auch die Expression der weiteren radiale-Gliazellenspezifischen Marker Vimentin und Glast wurde in mehr als der Hälfte der 473HD-positiven Zellen festgestellt. Unerwartet waren in der angereicherten Zellpopulation 25% βIII-Tubulin-positive postmitotische Neurone zu finden. Die Ergebnisse der immuncytochemischen Analysen der selektiv isolierten 473HD-positiven Zellpopulation sind in Tabelle 3 zusammengefasst.

Tab. 3: Ergebnisse der immuncytochemischen Analysen mit zelltypspezifischen primären Antikörpern an 473HD-selektierter Zellpopulation nach 2h *in vitro* (n = Anzahl unabhängiger Experimente)

| Zelltypspezifische Marker | Anteil (%) positiver Zellen in der isolierten 473HD- selektierten Zellpopulation | |
|---|---|---|
| Nestin | 62 ± 13 | (n=11) |
| RC2 | 85 ± 4.1 | (n=6) |
| Vimentin | 61 ± 5.8 | (n=10) |
| Glast | 55 ± 3.2 | (n=11) |
| βIII-Tubulin | 25 ± 7.1 | (n=11) |
| GFAP | 5.1 ± 3.9 | (n=7) |
| 473HD | 90 ± 6.9 | (n=11) |

Um die Eigenschaften der 473HD-positiven Zellen weiter zu beschreiben, wurde das Differenzierungsverhalten der nach dem Immunopanning isolierten 473HD-positiven Zellpopulation in serumhaltigen Medium auf Lamininsubstrat *in vitro* beobachtet. Dabei wurde nach dem zweiten Tag *in vitro* (2 div) eine immuncytochemische Analyse durchgeführt, die durch Anwendung von zelltypspezifischen Antikörpern die Aussage über Veränderungen in der Zusammensetzung der isolierten Zellpopulation *in vitro* erlaubte (Fig. 12).

Nach 2 Tagen in Kultur wies der größte Anteil der Zellen eine bipolare Morphologie auf (Fig. 12A) und war bis zu 82 ± 2,6% (n=4) βIII-Tubulin-positiv (Fig. 12B). Die überwiegende Zahl der 473HD-positiven radialen Gliazellen differenzierte also unter diesen Kulturbedingungen zu Neuronen. Dementsprechend nimmt der Anteil von Zellen, die radiale Gliamarker exprimieren, ab. So fiel der Anteil Glast-positiver Zellen von 55% auf 19% und Vimentin-positiver Zellen von 61% auf 48 %.

Die absteigende Tendenz zeigte sich auch für die Nestin-positiven Zellen, nach 2 div ging der Anteil dieser Zellen von 62% auf 6,5% zurück.

Das 473HD-Epitop exprimierten nach 2 div 25% der Zellen. Die Ergebnisse der immuncytochemischen Analysen mit zelltypspezifischen primären Antikörpern an der selektiv isolierten Zellpopulation nach 2 Tagen *in vitro* sind in Tab. 4 zusammengefasst.

Tab. 4: Veränderungen der Zusammensetzung der 473HD-positiven Zellpopulation nach 2div (n = Anzahl unabhängiger Elemente)

| Zelltypspezifische Marker | Anteil (%) positiver Zellen der isolierten 473HD-selektierten Zellpopulation nach 2div | |
|---|---|---|
| 473HD | 25 ± 4,4 | (n=4) |
| βIII-Tubulin | 82 ± 2,6 | (n=4) |
| Vimentin | 48 ± 5,2 | (n=4) |
| Glast | 19 ± 2,3 | (n=4) |
| Nestin | 6.5 ± 1,4 | (n=4) |
| GFAP | 16 ± 4 | (n=4) |
| O4 | 0 | (n=4) |

B. Neurosphärenbildung in serumfreiem Medium:

Zellen aus primär isolierten Einzelzellsuspensionen von E13 Vorderhirn und Zellen der selektiv isolierten 473HD-positiven Zellen aus dieser Einzelzellpopulation wurden unter Standardbedingungen (100.000 Zellen pro 1 ml Neurosphärenmedium mit bFGF und EGF) sieben Tage *in vitro* (7div) beobachtet (Fig. 13).

Dabei wurde festgestellt, dass 18 ± 4% der 473HD-positiven Zellen in der Lage waren, unter Standardbedingungen Neurosphären zu bilden. Die Neurosphärenbildung verlief jedoch im Vergleich zu Zellen aus einer primär isolierten Einzelzellsuspension wesentlich langsamer (Fig. 13).

Die Fähigkeit von selektiv isolierten 473HD-positiven Zellen zur Neurosphärenbildung wurde bei verschiedenen Zelldichten pro ml Medium beobachtet. So wurden zusätzlich zu den in den beiden vorhergehenden Absätzen beschriebenen Experimentbedingungen sog. Einzelzellklonierungen durchgeführt, bei denen aus selektiv isolierter Population ausgehend einer Suspension mit 10 Zellen/ml im statistischen Durchschnitt 1 Zelle pro Vertiefung in einer Mikrotiterplatte (96 well) in 100 μl Neurosphärenmedium mit EGF und bFGF ausplattiert wurde. Innerhalb von 7 div wurden von diesen Zellen Neurosphären gebildet. Unter diesen Bedingungen wurde eine stark beschleunigte Neurosphärenbildung bei geringerer Zelldichte im Vergleich zu den Standardkulturen beobachtet (Fig.14).

Es zeigte sich, dass bei geringer Zelldichte (1 Zelle/100 μl) ein Fünftel der Zellen (18 ± 4%, n=4) Neurosphären bilden können. Somit enthielt die 473HD-positive Zellpopulation mehr neurale Stammzellen als die primär isolierte Einzelzellsuspension bei E13 Vorderhirn, da im Vergleich mit nicht selektierten Zellen 4.5-fach mehr Neurosphären entstanden. Um zu überprüfen, ob diese Zellen selbsterneuerungsfähig sind, wurde aus primären Neurosphären eine Einzelzellsuspension hergestellt und unter identischen Kulturbedingungen die Bildung von sekundären Neurosphären beobachtet. 20,4 % der Zellen waren in der Lage, sekundäre Neurosphären zu bilden.

C. Differenzierungsanalyse der Neurosphärenkulturen: Neben der Fähigkeit zur Bildung der multizelluläre Neurosphären *in vitro* sind die neuralen Stammzellen auch durch ihren multipotenten Charakter gekennzeichnet. Dies äußert sich *in vivo* und *in vitro* durch Differenzierung dieser Zellen zu unterschiedlichen neuralen Zelltypen. Nachdem festgestellt wurde, dass zwischen 14% und 22% (n=4) der 473HD-positiven Zellen nach der selektiven Isolierung zur Neurosphärenbildung fähig sind, blieb offen, ob die Zellen aus diesen Neurosphären multipotenten Charakter aufweisen. Zur Beantworten dieser Frage wurde eine Differenzierungsanalyse mit primären Neurosphären durchgeführt. Die Bildung von Neuronen, Astrozyten, Oligodendrozyten und weiteren Vorläuferzellen wurde durch die zelltypspezifischen Antikörper nachgewiesen und der prozentuale Anteil aller Zellen quantitativ ermittelt. Die Differenzierungsanalyse wurde parallel an aus selektiv isolierten 473HD-positiven Zellen und primärer Einzelzellpopulation gebildeten Neurosphären durchgeführt. In Fig. 15 sind exemplarische fluoreszenzmikroskopischen Aufnahmen der verschiedenen Zelltypen dargestellt. Die dokumentierten Aufnahmen der Immunfärbungen lassen erkennen, dass sich aus primären Neurosphären unterschiedliche neurale Zelltypen differenzieren, unabhängig davon, aus welchen Zellen diese Neurosphären entstanden sind. Der quantitative Vergleich zeigte nur leichte Unterschiede.

Die Ergebnisse dieses Experiments (n=1) sind in der Tabelle 5 zusammengefasst.

Tab. 5: Vergleich der immuncytochemischer Analyse an ausdifferenzierten primären Neurosphären.

| Zelltypspezifische Marker | %-Anteil positiver Zellen in Neurosphären aus primärer Einzelzellpopulation | %-Anteil positiver Zellen in Neurosphären aus 473HD-selektierter Zellpopulation |
|---|---|---|
| βIII-Tubulin | 8,5 | 9,3 |
| GFAP | 4 | 5,9 |
| O4 | 6,1 | 2,4 |
| Glast | 6,2 | 13,1 |
| Nestin | 36,6 | 20,3 |
| RC2 | 8,9 | 13 |
| 473HD | 10,4 | 13,6 |

Es konnte somit gezeigt werden, dass Neurosphären, die aus der 473HD-positiven Zellpopulation entstehen, multipotent sind, da sowohl Neurone (Fig. 15A), als auch Astrozyten (Fig. 15D) und Oligodendrozyten (Fig. 15G) entstehen. Diese Eigenschaft gilt als stammzellspezifisch.

Beispiel 9: Nachweis des Mab 473HD-Epitops auf der Oberfläche humaner Zellen

A. Zellinien: Die humane Zelllinie WISH (ATCC Nr. CCL-25) wurde ursprünglich von humanem Amnionepithel abgeleitet. Dieser Zelltyp entstammt also dem Amnion, einer mit Fruchtwasser gefüllten Blase, welche den Embryo der Landwirbeltiere, insbesondere des Menschen, umhüllt.

Die Zellinie U-87MG (ATCC Nr. HTB-14) wurde aus einem menschlichen Hirntumor (Glioblastom) gewonnen (Ponten, J. und MacIntyre, E.H., Acta Path. Microbiol. Scandinav. 74:465-486 (1968)).

B. Zellkultur: Die U-87MG-Zellen wurden in alpha-MEM (Gibco (Invitrogen)), Karlsruhe, Deutschland) mit 10% (v/v) fötalem Kälberserum (Seromed, Berlin, Deutschland), und 0,1 % Gentamycin (Gibco) bei 37°C, 6 % (v/v) $CO_2$ kultiviert. Die WISH-Zellen wurden in RPMI (Gibco) mit 10 % (v/v) fötalem Kälberserum (Seromed) bei 37°C, 6% $CO_2$ gehalten.

Zur Anzucht der Zellen wurden Standard-Zellkultur-Petrischalen oder Kulturflaschen (NUNC, Wiesbaden, Deutschland) mit 100 μg/ml Polylysin (Sigma, München, Deutschland) in dd$H_2O$ 1 h bei 37°C vorinkubiert, zweimal mit dd $H_2O$ und einmal mit HBSS (Gibco) gewaschen und mit 10 ml der Zellsuspension mit 5x10^5 Zellen/ml beschickt. Das Medium wurde abhängig vom Volumen der Kulturflasche auf 20-40 ml aufgestockt und die Zellen bis zur Konfluenz kultiviert. Zur Subkultivierung wurde das Medium entfernt, der Zellrasen zweimal mit auf 37°C vorgewärmten HBSS gewaschen und 5 Minuten bei 37°C mit Trypsin-EDTA (Gibco) inkubiert. Die Trypsin-behandelten Zellen wurden in einem sanften Pipettenstrom vom Substrat gelöst, 5 Minuten bei 120 g (1000 rpm) zentrifugiert und in 10 ml Kulturmedium resuspendiert. Die Zellzahl wurde mit der Neugebauer-Zählkammer ermittelt und die Zellen wie oben beschrieben ausplattiert.

Für die Kultur auf Deckgläsern wurden runde Glasplättchen von 11 mm Durchmesser zunächst im Trockenschrank bei 130°C sterilisiert, 1 h bei 37°C im Brutschrank in einer Lösung von 100 $\mu$g/ml Polylysin (Sigma) inkubiert, zweimal in ddH$_2$O gewaschen und in Petrischalen oder 4-Napf-Platten (NUNC) überführt. Flüssigkeitsreste wurden mit einer Pasteurpipette unter Unterdruck abgesaugt und 5x10$^4$ Zellen in 50 $\mu$l (entspricht 10$^6$ Zellen/ml) ausplattiert. Die Kulturen wurden 1 h bei 37°C, 6% CO$_2$ im Brutschrank gehalten, um die Anheftung der Zellen an das Substrat zu erlauben und anschließend mit dem zugehörigen Kulturmedium geflutet. Die Kulturen wurden bis zur Konfluenz eines einschichtigen Zellrasens im Brutschrank gehalten und für immunzytologische Färbungen verwendet. Dies geschah in der Regel nach 2-3 Tagen.

C. Nachweis des 473-HD-Epitops durch Immunzytologie: Deckgläser mit weitgehend konfluenten Kulturen von U-87MG oder WISH-Zellen wurden der Kulturschale entnommen, zweimal mit 125 mM NaCl, 4,8 mM KCl, 1,3 mM CaCl$_2$-2H$_2$O, 1,2 mM MgSO$_4$-7H$_2$O, 1,2 mM KH$_2$PO$_4$, 5,6 mM D-Glukose (Sigma), 25 mM HEPES (Gibco) (folgend KRH genannt) gewaschen und mit 1 $\mu$g/ml des monoklonalen Antikörpers (Mab) 473HD in KRH, 10 mg/ml BSA (Sigma) (folgend KRH-BSA bezeichnet) für 30 min bei Raumtemperatur (RT) inkubiert. Anschließend wurde die Kultur zweimal mit PBS gewaschen und mit 4% (w/v) Paraform aldehyd in PBS (PFA) für 15 Minuten bei RT fixiert. Die Kulturen wurden zweimal mit PBS gewaschen und mit PBS, 0,1% (w/v) BSA für 10 min bei RT blockiert. Anschließend erfolgte die Inkubation mit dem Zweitantikörper. Hierbei handelte es sich um Fluorophor-derivatisierte (Cy2 oder Cy3) oder biotinylierte, affinitätsgereinigte anti-Ratten IgM-spezifische Zweitantikörper (Dianova, Hamburg). Wenn biotinylierte Zweitantikörper verwendet wurden, erfolgte die Entwicklung mit Fluorophor-gekoppeltem Streptavidin. Die Zweitantikörper wurden in KRH-BSA 1:200 - 1:500 verdünnt (dies muß Chargen-abhängig ausgetestet werden) und 30 Minuten mit den Kulturen inkubiert. Anschließend wurden die Kulturen zweimal mit PBS gewaschen und mit Immumount (Shandon, USA) eingedeckelt. Die Auswertung der fluoreszent markierten Zellen erfolgte am Axiphot oder am konfokalen Laser Scaning Mikroskop LSM510 Meta der Firma Zeiss. Sowohl in den U-87MG als auch in den WISH-Kulturen fand sich eine starke Substratkonditionierung mit Mab 473HD-positivem Material. Diese Substrat-Konditionierung legt nahe, dass das 473HD-Epitop in diesen Systemen auf einem in das Kulturmedium freigesetzten Chondroitinsulfat Proteoglykan exprimiert ist. Dieses Material konnte in dem konditionierten Medium der U-87MG und der WISH-Zelllinien auch durch enzyme-linked-immunosorbent assay (ELISA) und durch Western Blot mit dem Mab 473HD nachgewiesen werden. Ferner wurde beobachtet, dass 473HD-positive Signale mit der Oberfläche von bis zu 50% der WISH-Zellen assoziiert waren. Die Oberflächenfärbung zeigte die typischen Charakteristika einer membranständigen Färbung, wie sie auch an den neuralen Vorläuferzellen der Maus beobachtet worden war. I n Analogie zu den Versuchen, die zur Anreicherung der neuralen Vorläuferzellen geführt hatten, wurde eine Suspension von WISH Zellen hergestellt und für 2h im Brutschrank bei 37°C, 6% CO$_2$ inkubiert, um den Zellen nach der Trypsinisierung Gelegenheit zur Restitution der Zelloberflächenstrukturen zu geben. Die Zellsuspension wurde mit 1 $\mu$g/ml Mab 473HD in KRH-BSA inkubiert, zweimal durch Zentrifugation und Resuspension in HBSS gewaschen und daraufhin mit PBS 4% (w/v) PFA für 15 min bei RT fixiert. Anschließend wurden die Zellen abzentrifugiert, mit PBS, 0,1% (w/v BSA), für 15 min blockiert, zweimal mit PBS gewaschen und anschließend mit biotinylierten, anti-Ratten-IgG und -IgM in KRH-BSA inkubiert, zweimal in HBSS zentrifugiert und resuspendiert und nach der erfolgten Waschung mit Fluorophor-derivatisiertem Streptavidin entwickelt. Es zeigte sich, dass Mab 473HD-positive WISH-Zellen mit dieser Methode in der Petrischale angefärbt werden konnten. Diese Zellen können unterschiedlichen Sortierverfahren zugeführt werden, z.B. dem "fluorescence-activated cell sorter" (FACS).

## Patentansprüche

1. Verfahren zur Isolierung und Kultivierung von neuralen Stammzellen aus Vertebratengewebe, umfassend die Selektion der Stammzellen aus einer Einzelzellsuspension von Vertebratengewebe mittels wenigstens einem gegen DSD-1-Epitope gerichteten Antikörper (primärer Antikörper), der zur spezifischen Bindung mit dem DSD-1-Epitop befähigt ist, wobei das Vertebratengewebe kein humanes embryonales Gewebe ist.

2. Verfahren nach Anspruch 1, wobei

   (i) der primäre Antikörper wenigstens eine Bindungsstelle für ein spezifisches sekundäres Agens aufweist; und/oder
   (ii) die Vertebraten Vögel, Fische oder Säuger, insbesondere Mensch, Maus oder Ratte, sind; und/oder
   (iii) die Einzelzellsuspension aus embryonalem, postnatalem und/oder adultem Gewebe, insbesondere aus Nervengewebe oder Gehirn hergestellt wird.

**3.** Verfahren nach Anspruch 1 oder 2, wobei der primäre Antikörper ein monoklonaler oder polyklonaler, vorzugsweise ein monoklonaler Antikörper ist.

**4.** Verfahren nach einem oder mehren der Ansprüche 1-3, wobei der primäre Antikörper

(i) ein Epitop für einen sekundären Antikörper aufweist, und/oder
(ii) ein humaner, muriner, Ratten-, Kaninchen-, Ziegen- oder Schweineantikörper ist.

**5.** Verfahren nach Anspruch 4, wobei der primäre Antikörper durch Immunisieren von Ratten mit einer Präparation, umfassend Glykoproteine, Proteoglycane und Glycoproteinfraktionen adulter Mäusehirnmembrane, erhältlich ist, und/oder Epitope für einen sekundären Antikörper anti-Ratte aufweist, welcher die Subklassen IgG, IgA, IgE, IgM oder die leichten Ketten $\chi$ und $\lambda$ erkennt.

**6.** Verfahren nach Anspruch 5, wobei der sekundäre Antikörper anti-Ratte Ig CY3 oder anti-Ratte Ig biot+ Streptavidin FITC ist.

**7.** Verfahren nach Anspruch 5 oder 6, wobei der primäre Antikörper der monoklonale Antikörper 473HD, der von den Hybridomazelle DSM ACC2671 produziert wird, ist.

**8.** Verfahren nach einem oder mehreren der Ansprüche 2 bis 7 wobei

(i) der primäre Antikörper durch Wechselwirkung mit dem wie in Anspruch 4(i) definierten sekundären Antikörper oder durch eine andere adäquate Kupplungstechnik, welche die Spezifität des primären Antikörpers nicht verändert, vorzugsweise durch kovalente Vernetzung an ein Trägermaterial gebunden ist, und/oder
(ii) durch spezifische Bindung neuraler Stammzellen aus der Einzelzellsuspension an den primären Antikörper eine Isolierung dieser Zellen aus der Zellkultur erfolgt, die nicht gebundene Zellen entfernt werden und anschließend die über den primären Antikörper und ggf. den sekundären Antikörper an das Trägermaterial gebundenen Zellen durch Inkubation mit einer geeigneten Protease oder ein anderes adäquates Verfahren vom Trägermaterial abgelöst werden, und/oder
(iii) Säugergewebe verwendet wird und das Säugergewebe embryonal, postnatal oder adult ist, wobei das Säugergewebe bevorzugt vom Menschen oder von Nagern stammt, und/oder
(iv) das Verfahren *in vitro* erfolgt.

**9.** Verfahren nach einem oder mehreren der Ansprüche 1 bis 8, wobei

(i) das Vertebratengewebe, vorzugsweise durch Inkubation in Gegenwart einer Protease, bevorzugt einer Serin-Protease, besonders von Trypsin, in eine Einzelzellkultur überführt wird, und insbesondere die Zellen der Einzelzellkultur nach Abschluss der Behandlung mit einer Protease vor Beginn der Selektion 0,5-6 h, bevorzugt 1-4 h, besonders bevorzugt 1-2 h, in einem geeigneten Medium oder Puffer inkubiert werden; und/oder wobei
(ii) die isolierten Stammzellen zur Kultivierung zu Neurosphären verwendet werden und diese Kultivierung in geeigneten Kultivierungsmedien geschieht.

**10.** Verwendung des monoklonalen Antikörpers 473HD, welcher von der Hybridomzelllinie DSM ACC2671 produziert wird, zur Gewinnung von neuralen Stammzellen.

**Claims**

**1.** A process for the isolation and cultivation of neural stem cells from vertebrate tissue, comprising the selection of the stem cells from a single cell suspension of vertebrate tissue by means of at least one antibody raised against DSD-1 epitopes (primary antibody) that is capable of specific bonding to said DSD-1 epitope, provided that said vertebrate tissue is not human embryonic tissue.

**2.** The process according to claim 1, wherein

(i) said primary antibody has at least one binding site for a specific secondary agent; and/or
(ii) said vertebrates are birds, fish or mammals, especially humans, mice or rats; and/or
(iii) said single-cell suspension is prepared from embryonic, postnatal and/or adult tissue, especially from nerve tissue or brain.

3. The process according to claim 1 or 2, wherein said primary antibody is a monoclonal or polyclonal antibody, preferably a monoclonal antibody.

4. The process according to one or more of claims 1 to 3, wherein said primary antibody

   i) has an epitope for a secondary antibody; and/or
   ii) is a human, murine, rat, rabbit, goat or pig antibody.

5. The process according to claim 1, wherein said primary antibody is obtainable by immunizing rats with a preparation comprising glycoproteins, proteoglycans and glycoprotein fractions of adult murine brain membranes, and/or has epitopes for a secondary anti-rat antibody that recognizes the subclasses IgG, IgA, IgE, IgM or the light chains $\chi$ and $\lambda$.

6. The process according to claim 5, wherein said secondary antibody is anti-rat Ig CY3 or biotinylated anti-rat Ig + streptavidin-FITC.

7. The process according to claim 5 or 6, wherein said primary antibody is the monoclonal antibody 473HD produced by the hybridoma cell DSM ACC2671.

8. The process according to one or more of claims 2 to 7, wherein

   (i) said primary antibody is bound to a support material by interaction with the secondary antibody as defined in claim 4(i) or by another adequate coupling technique which does not change the specificity of the primary antibody, preferably by covalent cross-linking; and/or
   (ii) isolation of neural stem cells from the cell culture is effected by specific binding of these cells from said single cell suspension to said primary antibody, the unbound cells are removed, and the cells bound to the support material through said primary antibody and, if applicable, said secondary antibody are subsequently detached from the support material by incubation with a suitable protease or another adequate method; and/or
   (iii) mammal tissue is used, and said mammal tissue is embryonic, postnatal or adult, wherein said mammal tissue is preferably derived from humans or rodents; and/or
   (iv) the process is effected in vitro.

9. The process according to one or more of claims 1 to 8, wherein

   (i) said vertebrate tissue is converted to a single cell culture preferably by incubation in the presence of a protease, preferably a serine protease, especially trypsin, and in particular, the cells of said single cell culture are incubated in a suitable medium or buffer for 0.5-6 hours, preferably for 1-4 hours, more preferably for 1-2 hours, after the treatment with a protease is completed before the beginning of the selection, and/or wherein
   (ii) the isolated stem cells are used for culturing neurospheres, the culturing being performed in appropriate culture media.

10. Use of the monoclonal antibody 473HD produced by the hybridoma cell line DSM ACC2671 for obtaining neural stem cells.

**Revendications**

1. Procédé pour isoler et cultiver des cellules souches neurales à partir de tissu de vertébrés, comprenant la sélection des cellules souches à partir d'une suspension monocellulaire de tissu de vertébrés, à l'aide d'au moins un anticorps dirigé contre des épitopes DSD-1 (anticorps primaire), qui est à même de se lier d'une manière spécifique à l'épitope DSD-1, le tissu de vertébrés n'étant pas un tissu embryonnaire humain.

2. Procédé selon la revendication 1, dans lequel

   (i) l'anticorps primaire comprend au moins un site de liaison pour un agent secondaire spécifique ; et/ou
   (ii) les vertébrés sont des oiseaux, des poissons ou des mammifères, en particulier l'homme, la souris ou le rat ; et/ou

(iii) la suspension monocellulaire est fabriquée à partir d'un tissu embryonnaire, post-natal et/ou adulte, en particulier d'un tissu nerveux ou du cerveau.

**3.** Procédé selon la revendication 1 ou 2, dans lequel l'anticorps primaire est un anticorps monoclonal ou polyclonal, de préférence un anticorps monoclonal.

**4.** Procédé selon l'une ou plusieurs des revendications 1 à 3, dans lequel l'anticorps primaire

(i) comprend un épitope pour un anticorps secondaire, et/ou
(ii) est un anticorps humain, murin, de rat, de lapin, de chèvre ou de porc.

**5.** Procédé selon la revendication 4, dans lequel l'anticorps primaire peut être obtenu par immunisation de rats avec une préparation comprenant des glycoprotéines, des protéoglycanes et des fractions glycoprotéiques de membranes de cerveau de souris adulte, et/ou comprend des épitopes pour un anticorps secondaire anti-rat, lequel reconnaît les sous-classes IgG, IgA, IgE, IgM ou les chaînes légères $\chi$ et $\lambda$.

**6.** Procédé selon la revendication 5, dans lequel l'anticorps secondaire est une Ig CY3 anti-rat ou une Ig biot+ streptavidine FITC anti-rat.

**7.** Procédé selon la revendication 5 ou 6, dans lequel l'anticorps primaire est l'anticorps monoclonal 473HD, qui est produit à partir de la cellule d'hybridome DSM ACC2671.

**8.** Procédé selon l'une ou plusieurs des revendications 2 à 7, dans lequel

(i) l'anticorps primaire est lié à un matériau support par une interaction avec l'anticorps secondaire tel que défini dans la revendication 4(i), ou par une autre technique de couplage adéquate, qui ne modifie pas la spécificité de l'anticorps primaire, de préférence par une réticulation covalente, et/ou
(ii) par une liaison spécifique, à l'anticorps primaire, de cellules souches neurales provenant de la suspension monocellulaire, on procède à un isolement de ces cellules à partir de la culture cellulaire, on élimine les cellules non liées, puis, par incubation avec une protéase appropriée ou par un autre procédé adéquat, on détache du matériau support les cellules liées au matériau support par l'intermédiaire de l'anticorps primaire et/ou de l'anticorps secondaire, et/ou
(iii) on utilise un tissu de mammifère, et le tissu de mammifère est embryonnaire, post-natal ou adulte, le tissu de mammifère provenant de préférence d'humains ou de rongeurs, et/ou
(iv) le procédé est mis en oeuvre *in vitro*.

**9.** Procédé selon l'une ou plusieurs des revendications 1 à 8, dans lequel

(i) le tissu de vertébrés est, de préférence par incubation en présence d'une protéase, de préférence une sérine-protéase, provenant de préférence de la trypsine, est converti en une culture monocellulaire, et en particulier les cellules de la culture monocellulaire sont incubées, après la fin du traitement avec une protéase avant le début de la sélection, pendant 0,5 à 6 h, de préférence pendant 1 à 4 h, d'une manière particulièrement préférée pendant 1 à 2 h, dans un milieu ou un tampon approprié ; et/ou dans lequel
(ii) les cellules souches isolées sont utilisées pour la culture de neurosphères, cette culture ayant lieu dans des milieux de culture appropriés.

**10.** Utilisation de l'anticorps monoclonal 473HD, produit par la lignée de cellules d'hybridome DSM ACC2671, pour obtenir des cellules souches neurales.

Fig.1

Fig.2

Fig.3

Fig.4

Fig.5

Fig.6

Fig.7

Fig.8

| Zelltypspezifische Marker | Anteil (%) positiver Zellen in der akut dissoziierten Zellpopulation |
|---|---|
| 473HD | 21 ± 4,9 |
| Nestin | 46 ± 9,8 |
| RC2 | 67 ± 9,3 |
| Vimentin | 42 ± 7,9 |
| Glast | 40 ± 12 |
| ßIII-Tubulin | 30 ± 8,4 |
| GFAP | 3 ± 1,5 |

21%
473HD+ Zellen

Fig.9

Fig.10

Fig.11

Fig.12

Fig.13

Fig. 1 4

Fig.15

**Immunopanning**

☐ 473HD+
Zellen

**473HD-selektierte
Zellpopulation**

**2 h In vitro**

90 % 473HD+
85 % RC2+
61 % Vimentin+
55 % Glast+
62 % Nestin+
25 % ßIII-tub. +
5 % GFAP +

**2 d In vitro**

25 % 473HD+
48 % Vimentin+
19 % Glast+
6 % Nestin+
82 % ßIII-tub. +
16 % GFAP +

Fig.16

**Immunopanning**

☐ 473HD+
Zellen

473HD-selektierte
Zellpopulation

Neurosphärenkulturen
7 d *in vitro*

**Differenzierungsanalyse der Neurosphärenkulturen**

10.4 % 473HD+
36.6 % Nestin+
8.9 % RC2+
6.2 % Glast+
8.5 % ßIII-tub. +
6.1 % O4+
4 % GFAP +

13.6 % 473HD+
20.3 % Nestin+
13 % RC2+
13.1 % Glast+
9.3 % ßIII-tub. +
2.4 % O4+
5.9 % GFAP +

Fig.17

**EP 1 771 549 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0052143 A
- US 5928947 A
- US 5654183 A
- US 5672499 A
- US 5693482 A
- DE 10242337 A
- US 20020168767 A
- WO 9520397 A
- JP 61282399 A
- JP 7631376 A

**In der Beschreibung aufgeführte Nicht-Patentliteratur**

- **TEMPLE, S.** *Nature,* 1989, vol. 340, 471-473
- **CATTANEO, E. et al.** *Nature,* 1990, vol. 347, 762-765
- **KILPATRICK, T. J. et al.** *Neuron,* 1993, vol. 10, 255-265
- **REYNOLDS, B. A. et al.** *J. Neurosci.,* 1992, vol. 12, 4565-4574
- **LOIS, C. et al.** *Science,* 1993, vol. 255, 1707-1710
- **MCKAY, R. D.** *Science,* 1997, vol. 276, 66-71
- **RAO, M. S.** *Anat. Rec.,* 1999, vol. 257, 137-148
- **GAGE, F. H.** *Science,* 2000, vol. 287, 1433-1438
- **ZAPPONE, M. et al.** *Development,* 2000, vol. 127, 2367-2382
- **WOLPERT, L. et al.** *Dev. Genet.,* 1994, vol. 15, 485-490
- **ALTMAN ; BRIVANLOU.** *Int. Rev. Cytol.,* 2001, vol. 203, 447-482
- **TROPEPE, V. et al.** *Dev. Biol.,* 2000, vol. 2-08, 166-188
- **BROWN, M. et al.** The Developing Brain. Oxford Univ. Press I nc, 2001
- **LANDER, A. D.** *Curr. Opin. Neurobiology,* 1993, vol. 3, 716-723
- **BANDTLOW ; ZIMMERMANN.** *Phys. Rev.,* 2000, vol. 80, 1267-1290
- **FAISSNER, A. et al.** *J. Cell. Biol.,* 1994, vol. 126, 783-799
- **GARWOOD, J. et al.** *J. Neurosci,* 1999, vol. 19, 51-64
- **GARWOOD, J. et al.** *J. Biol. Chem.,* 2003, vol. 278, 24164-14173
- **CLEMENT, A. M. et al.** *J. Biol. Chem.,* 1998, vol. 273, 28444-28453
- **FAISSNER, A.** *Soc. Neurosci. Abstr.,* 1988, vol. 14, 920
- **CLEMENT, A.M. et al.** *J. Biol. Chem.,* 1998, vol. 273, 28444-28453
- **HIKINO, M et al.** *Biol. Chem.,* 2003, vol. 278, 43744-43754
- **BAO, X. et al.** *J. Biol. Chem.,* 2005, vol. 280, 9180-9191
- **ITO, Y. et al.** *Glycobiology,* 2005, vol. 15, 593-603
- **HEYMAN et al.** *Dev. Dynam.,* 1995, vol. 204, 301-315
- **WINTERGERST, E.S. et al.** *Int. J. Dev. Neurosci.,* 1996, vol. 14, 249-256
- **MEYER-PUTTLIZ, B. et al.** *J. Neurochem.,* 1995, vol. 65, 2327-2337
- **ALLENDOERFER, K. L. et al.** *Dev. Biology,* 1999, vol. 211, 208-219
- **KRUEGER, N. X. ; SAITO, H.** *Proc. Natl. Acad. Sci. USA,* 1992, vol. 89, 7417-7421
- **CANOLL, P. D. et al.** *Brain Res.,* 1993, vol. 75, 293-298
- **MAUREL, P. et al.** *Proc. Natl. Acad. Sci. USA,* 1994, vol. 91, 2512-2516
- **ENGEL, M. et al.** *J. Comp. Neurobiol.,* 1996, vol. 366, 34-43
- **MEYER-PUTTLIZ, B. et al.** *J. Comp. Neurobiol.,* 1996, vol. 366, 44-54
- **IVANOVA, A. et al.** *Gene Expression Patterns,* 2004, vol. 4, 161-166
- **KABOS, P. et al.** *Biochem. Biophys. Res. Comm.,* 2004, vol. 318, 955-963
- **CANOLL, P. D. et al.** *J. Neurosci. Res.,* 1996, vol. 44, 199-219
- **ENGEL, M. et al.** *J. Comp. Neurol.,* 1996, vol. 366, 34-43
- **MEYER-PUTTLIZ, B. et al.** *J. Comp. Neurol.,* 1996, vol. 366, 44-54
- **SCHNÄDELBACH, O. et al.** *Glia,* 1998, vol. 23, 99-109
- **GARWOOD, J. et al.** *Neuroscience,* 2001, vol. 19, 51-64
- **DOBBERTIN, A. et al.** *Mol. Cell. Neurosci.,* 2003, vol. 24, 951-971

- **SAKURAI, T. et al.** *J. Neurosci. Res.,* 1996, vol. 43, 694-706
- **MAEDA, N. et al.** *Development,* 1996, vol. 122, 647-658
- **MILEV, P. et al.** *J. Biol. Chem.,* 1995, vol. 270, 24650-24653
- **MILEV, P. et al.** *J. Biol. Chem.,* 1988, vol. 273, 6998-7005
- **FAWCETT, J. W. et al.** *Brain Res. Bull.,* 1999, vol. 49, 377-391
- **BOVOLENTA, P.** *Prog. Neurobiol.,* 2000, vol. 61, 113-132
- **HARTMANN, U. et al.** *Matrix Biol.,* 2001, vol. 20, 23-35
- **FAISSNER, A.** *Neurosci. Abstr.,* 1988, vol. 14, 920
- **ROSZINSKI, S. et al.** *J. Radiat. Oncol. Biol. Phys.,* 1991, vol. 20, 1273-1280
- **WLODARSKI, K. et al.** *Calcif. Tissue Res.,* 1970, vol. 5, 70-79
- **FIORINI, S. et al.** *Endocrinology,* 2003, vol. 144, 3359-3367
- **PONTEN, J. ; MACINTYRE, E.H.** *Acta Path. Micro-biol. Scandinav.,* 1968, vol. 74, 465-486
- **WENDEL, C.** Immuncytologische, immunchemische und funktionelle Untersuchungen zu RPTP-zeta/beta Expression in humanen Tumorzellen. *Diplomarbeit, Fakultät für Biologie der Ruhr-Universität Bochum,* 2005
- **HUTTNER, W. B. ; BRAND, M.** *Neurobiol.,* 1997, vol. 7, 29-39
- **MALATESTA, P. et al.** *Development,* 2000, vol. 127, 5253-5263
- **HARTFUSS, E. et al.** *Dev. Biol.,* 2001, vol. 229, 15-30
- **RAKIC, P.** *Science,* 1972, vol. 183, 425-427
- **HATTEN; M.E. et al.** *Annu. Rev. Neurosci.,* 1999, vol. 22, 511-539
- **ANDERSON, S. A. et al.** *Science,* 1997, vol. 278, 474-476
- **PEARLMAN, T. et al.** *Genes Dev.,* 1998, vol. 9, 769-782
- **TAN, S. S. et al.** *Neuron,* 1998, vol. 21, 295-304
- **DAVIS, A. A. ; TEMPLE, S.** *Nature,* 1994, vol. 372, 263-266
- **MAYER-PROSCHEL, M. et al.** *Neuron,* 1997, vol. 15, 773-785
- **MUJATABA, T. et al.** *Dev. Biol.,* 1999, vol. 214, 113-127
- **VOIGT, T.** *J. of Comparative Neurobiol.,* 1989, vol. 289, 74-88
- **EDWARDS, M. A. et al.** *Neurosci,* 1990, vol. 36, 12-144
- **CULICAN, S. M. et al.** *J. Neurosci.,* 1990, vol. 10, 684-692
- **HARTFUSS, E. et al.** *Developmental Biol.,* 2001, vol. 229, 15-30
- **GARWOOD, J. et al.** *J. Neurosci.,* 1999, vol. 19, 3888-3899
- **BARRES, B. A. et al.** *Cell,* 1992, vol. 70, 31-46
- **BARRES, B.A. et al.** *J. Cell Biol.,* 1999, vol. 147, 1123-1128
- **HARTFUSS, E. et al.** *Soc. Neurosci. Meet,* 1999, vol. 25, 522
- **NOCTOR et al.** *Nature,* 2001, vol. 409, 714-720
- **MALATESTA, P. et al.** *Neuron,* 2003, vol. 37, 751-764
- **MIYATA, T. et al.** *Neuron,* 2001, vol. 31, 727-741
- **TAMAMAKI, N. et al.** *J. Neurosci. Res.,* 2001, vol. 41, 51-60
- **TEMPLE, S.** *Nature,* 2001, vol. 414, 112-117
- **TROPEPE et al.** *Dev. Biol.,* 1999, vol. 208, 166-188
- **HIRN et al.** *Brain Res.,* 1981, vol. 214, 433-439
- **NORONHA, A. B. et al.** *Brain Res.,* 1986, vol. 385, 237-244
- **KRUSE, J. et al.** *Nature,* 1985, vol. 311, 153-155
- **FAISSNER, A.** *Soc. Neurosci. Abstr,* 1988, vol. 14, 920
- **FAISSNER, A.** *Soc. Neurosci Abstr.,* 1988, vol. 14, 920
- **BIGNAMI, A. et al.** *Brain Res.,* 1972, vol. 43, 429-435
- **LEVITT, P. ; RAKIC, P.** *Comp. Neurol.,* 1980, vol. 193, 815-840
- **DAHL D. et al.** *J. Comp. Neurol.,* 1985, vol. 239, 75-88
- **LEE, M. K. et al.** *Cell Motil. Cytoskeleton,* 1990, vol. 17, 118-132
- **SOMMER I. ; SCHACHNER, M.** *Dev. Biol.,* 1984, vol. 83, 311-327
- **MISSION, J. P. et al.** *Dev. Brain Res.,* 1988, vol. 44, 95-108
- **EDWARDS, M. A. et al.** *Neurosci.,* 1990, vol. 36, 12-144
- **FREDERIKSEN, K.** *J. Neurosci.,* 1988, vol. 8, 1144-1151
- **LENDAHL, U. ; MCKAY, R. D. G.** *Trends Neurosci.,* 1990, vol. 13, 132-137
- **MISSION, J. P et al.** *Dev. Brain,* 1988, vol. 44, 95-108
- **SHIBATA, T. et al.** *J. Neurosci.,* 1997, vol. 17, 9212-9219
- **PIXLEY, S. K. ; DE VELLIS, J.** *Brain Res.,* 1984, vol. 317, 201-209
- **MEYER, S. A. et al.** *J. Neurosci. Res.,* 1989, vol. 24, 251-259

- **MONZON-MAYOR, M. et al.** *J. Comp. Neurol.,* 1990, vol. 295, 569-579
- **OUDEGA, M. ; MARANI, E.** *J. Anat.,* 1991, vol. 179, 97-114
- **TEMPLE, S.** *Nature,* 2000, vol. 414, 112-117
- **QIAN, X. et al.** *Neuron,* 2000, vol. 28, 69-80
- **PONTEN, J. ; MACLNTYRE, E.H.** *Acta Path. Microbiol. Scandinav.,* 1968, vol. 74, 465-486